# EUROPEAN PATENT SPECIFICATION

(11) **EP 2 219 577 B1**
(45) Date of publication and mention of the grant of the patent: **30.07.2014**
(21) Application number: 08860061.4
(22) Date of filing: 03.12.2008
(51) Int. Cl.: A61F 13/15, A61F 13/495, A61F 13/512

(54) **ABSORBENT ARTICLE WITH COMPOSITE SHEET COMPRISING ELASTIC MATERIAL**
SAUGFÄHIGER ARTIKEL MIT ZUSAMMENGESETZTER PLATTE AUS EINEM ELASTISCHEN MATERIAL
ARTICLE ABSORBANT AVEC FEUILLE COMPOSITE COMPRENANT UN MATÉRIAU ÉLASTIQUE

(30) Priority: 13.12.2007 US 13493
(43) Date of publication of application: 25.08.2010
(73) Proprietor: The Procter & Gamble Company, Cincinnati, OH 45202 (US)
(72) Inventor: PONOMARENKO, Ekaterina, Anatolyevna, 65814 Bad Soden (DE); BROWN, Tina, Cincinnati Ohio 45241 (US); UNGER, Alexander, Eberhard, 65779 Kelkheim (DE); BERK, Alexander, 60316 Frankfurt/main (DE); SCHMIDT, Mattias, 65510 Idstein (DE); BROWN, Darrell, Ian, Mason Ohio 45040 (US); BLANCA, Arizti, 60325 Frankfurt/m (DE)
(74) Representative: Borbach, Markus
(86) International application number: PCT/IB2008/055070
(87) International publication number: WO 2009/074922

(56) References cited:
- EP-A- 1 803 430
- WO-A-99/25283
- US-A- 5 576 090
- US-A1- 2002 049 419
- US-A1- 2003 181 882
- US-B1- 6 258 196
- US-B1- 6 482 191

## Description

### FIELD OF THE INVENTION

This invention is directed to a process to make a specific composite sheet, useful as or in a topsheet with one or more openings herein, said sheet having specific elasticized regions with improved wrinkle profile and to absorbent articles comprising such specific (barrier) composite sheet materials.

### BACKGROUND OF THE INVENTION

It is well known that fecal material is often difficult to remove from the skin of the user, in particular from sensitive skin such as of young babies and such as the skin around the genitals. Moreover, it is well known that fecal material on the skin can cause irritation and redness of the skin and sometimes even dermatitis of the skin.

One of the solutions to reduce the fecal material on the skin is to provide a means to isolate the fecal material immediately after discharge, away from the skin. For example, diapers with an elasticized topsheet with an opening also referred to as anal and/ or genital cuff, through which the feces can pass to a void space between the topsheet and the absorbent core, have been developed. The fecal material is then stored underneath this topsheet, away from the skin. It may be particularly beneficial that such a topsheet is made of a material having barrier properties

US 2003/181882 A1 discloses an absorbent article having a substantially oblong shape and comprising a liquid permeable topsheet, a liquid impermeable backsheet, and a liquid retentive absorbent member interposed between the topsheet and the backsheet is disclosed. The topsheet is a bulky sheet which exhibits elastomeric behavior as a whole and comprises a first layer disposed on the side of a wearer and a second layer disposed on the side of the absorbent member. The first layer and the second layer are partially bonded together at joints in a prescribed pattern. The first layer has protrusions on the side of a wearer in portions other than the joints. The second layer is made of a material exhibiting elastomeric behavior. The topsheet is fixed in an extended state in at least one of the longitudinal direction and the width direction of the absorbent article.

US-B1-6 258 196 discloses a sintered porous composite sheet comprising an A/B-component layer in which the A-component layer is easily fusible and the B-component layer has a relatively higher thermal stability as compared with the A-component layer. The layers are superposed and sintered. The A-component layer comprises a hydrophilic material, the B-component layer comprises a hydrophobic material, and the A-component and B-component may include a common material comprising an easily fusible material. The porous composite sheet is air and moisture permeable, and is readily adaptable for use in sanitary and medical applications.

US 2002/049419 A1 discloses an absorbent article having a liquid permeable surface layer, a backing sheet, and an absorbent layer interposed between the surface layer and the backing sheet. The surface layer includes a liquid permeable base and a layer of continuous filaments extending substantially in one direction and disposed on the surface of the base. The continuous filament layer is fixed to the base at a plurality of fixing portions which are spaced apart in the filament extending direction by a given pitch, to thereby form loop portions of the continuous filaments between adjacent fixing portions. The loop portions are raised toward the surface side.

WO 99/25283 A1 discloses a disposable absorbent article having a liquid pervious topsheet, a backsheet joined to the topsheet, and a fecal management member positioned between the topsheet and the backsheet. The fecal management member having a pattern of arcuate portions.

EP-A-1 803 430 discloses disposable absorbent article structure such that an article body is composed of at least a top sheet arrange in a front face side, a back sheet arranged in a back face side and an absorber interposed between both of top sheet and back sheet, and a feces pocket is provided In a hip contact portion of the article body, wherein an opening portion of the feces pocket is covered with a mesh sheet.

US-A-5 576 090 discloses an expandable sheet elastic complex which consists of an elastic body sheet and a sheet backing material which is mounted on either one or both sides of it, these two members being bonded together along multiple, oblong, mutually parallel bonding sections, and in which multiple, mutually parallel channels are formed between the two members because the width of the sheet backing material between mutually adjacent bonding sections is greater than that of the elastic body sheet. The sheet backing material does not accept more than the minimum constraint of the elastic body sheet. The disclosure also provides a process for manufacturing this sheet elastic complex.

US-B1-6 482 191 discloses an absorbent article having a void space for receiving bodily exudates comprises an elongate slit opening in communication with the void space. The elongate slit opening includes elasticated region disposed along longitudinal side edges of the slit opening which maintain longitudinal and lateral alignment and Z-direction proximity with a point of discharge on a wearer during use. The elasticated regions contain stored elastic energy producing tensile forces which are distributed laterally away from the slit opening enabling the edges to deflect and allow bodily exudates to pass through the slit opening to the void space.

The inventors have found that often materials that provide a good barrier are not very comfortable in use. The inventors furthermore found that these barrier materials are sometimes difficult to elasticize, or that it is difficult to provide comfortable elastic regions in such materials. The inventors found that it may be desirable that the topsheet, also referred to as anal and/ or genital cuff, but also other elasticsed components have softer elastic regions for the sensitive (baby) skin. It has been found that it is important that the wrinkles, caused by the elastic regions, leave less pressure marks. It has been found that the wrinkles should thereto be very uniform. However, it has been found to be difficult to produce elasticized articles with uniform elastics at high speed, and in particular when the elastics are applied in a curvilinear pattern and/ or when the elastics are applied under high strain, and/ or when the elasticized material is thick or stiff, such as may be the case with high barrier materials.

The inventors found that with prior art processes, whereby the elastic material is merely attached in stretch state to a nonwoven with adhesives, the attachment of the elastic material in stretched state may result in irregular attachment areas and subsequently irregular wrinkles.

The inventors have now found an improved process to provide comfortable elasticized regions in topsheet with one or more openings, and they have found an improved plasticized composite sheet material useful in or as such topsheets and useful in absorbent articles. In said improved process of the invention, a first sheet is submitted to a patterning step to form troughs, which are then simultaneously attached by application of gentle pressure to a stretched clastic material, i.e. before the plasticized region is allowed to relax and form wrinkles. The resulting patterned, wrinkled composite sheet material has an improved wrinkle pattern that is softer and/ or more comfortable for the user. By patterning the first sheet with troughs and attaching these, or part thereof, to an elastic material prior to relaxation of the elastics, the subsequently wrinkle formation and wrinkle pattern can be controlled to have the desired uniformity.

### SUMMARY OF THE INVENTION

The present invention relates to a process for making a composite sheet (10) useful for an absorbent article, said composite sheet (10) comprising a wrinkled, patterned clasticized region (12a or 12b) that comprises a patterned first sheet (13) and an Mastic material (15), said composite sheet (10) being obtainable by:
a) obtaining a first sheet (13) with a Y-direction and X-dircetion;
b) obtaining an elastic material (15) that is at least partially stretched, having at least an average longitudinal direction of stretch Y (e.g. substantially along said Y-direction of said sheet);
c) positioning said at least partially stretched elastic material (15) adjacent said first sheet (13) to obtain a combined material and simultaneously or subsequently submitting the combined material, or part thereof, to a patterning, pressure-applying step to obtain a patterned combined material, comprising a patterned first sheet (13) comprising troughs (16);
d) simultaneously or subsequent to step c) attach the thus formed troughs (16), or part thereof, of the patterned first sheet (13) to said elastic material (15), to thus obtain a stretched composite sheet (10) comprising a patterned elasticized region;
e) relaxing the composite sheet (10) of step d) to obtain a composite sheet (10) comprising a wrinkled, patterned elasticized region (12), comprising wrinkles with peaks (21) and valleys (22), said composite sheet (10) comprising one or more openings to receive fecal material and comprising said step c ii) whereby said first sheet (13) and elastic material (15) are positioned adjacent one another prior to or simultaneously with said patterning, pressurizing step and whereby said step c ii) and d) are performed simultaneously.

Whilst the process of the present invention may be used to make any composite sheet (10) comprising an clastic material and a first sheet (13), or comprising even additional sheet materials, the process is in particular useful to make composite sheets (10) that have (and the invention relates to composite sheets that have): a barrier first sheet, as described herein; a thick or stiff first sheet, having the bending rigidity as described herein; curved or angled elastics e.g. curvilinear elastics; elasticized regions (12), whereby adhesion of the elastic material and first sheet (13) is achieved by application of pressure and optionally heat (and for example without adhesive present); and/ or elastic material applied under high strain.

The process may for example be used to make composite sheets (10) that comprise (and the invention relates to composite sheets that have) a first sheet (13) that is a nowoven sheet, comprising one or more nonwoven layers that is/ are (each) a laminate of one or more spunbond and one or more meltblown webs; and/ or a first sheet (13), having the preferred hydrostatic head values and/ or bending rigidity values, described herein.

In one embodiment, the surface of the first sheet (13) that is not facing the clastic material is pressurized by (and possibly directly contacted by) a first patterning tool's surface having raised portions and the opposite surface of the first sheet (13), which faces the elastic material, is pressurized ( and preferably indirectly contacted) by a second, non-mating surface of a tool, for example an even surface; thus, said first tool's surface (31) may be the surface of a patterned roll with raised portions and the second tool's surface being the surface of an anvil roll with non-mating raised portions, or preferably without raised portions, e.g. having an even surface.

The troughs of the first sheet (13) (i.e. the portions of the first sheet (13) that form the troughs) is typically compacter, having a higher density, than the portions of the first sheet (13) not forming the troughs, (e.g. the portions of the first sheet between neighboring troughs (16), including the crests (17) and optionally portions of the first sheet (13) that are not patterned and/ or that are not pressurized by the patterning tool).

It has furthermore been found that the provision of a pattern of troughs in the first sheet (13) and selectively attaching these troughs to the elastic material allows very effective attachment of the first sheet (13) to a minimum amount of elastic material (whilst this is in stretched state), and thus resulting in a minimum of elastic material that remains in stretched state after relaxation of the composite sheet. Thus, optimum strength of the attachment of the elastic material (15) to the first sheet (13) can be achieved, whilst at the same time optimum elasticity can be maintained.

In one embodiment herein, the invention provides an absorbent article comprising a composite sheet (10) that comprises a wrinkled and patterned elasticized region (12), said region containing a first sheet (13) and an elastic material (15), said elasticized region (12) having a residual strain of less than 30%, preferably less than 20%, or between 2% and 20%, and in one embodiment herein when a second sheet (14) is present (as described herein), having a peel force of at least 1.4 N or at least 2.0 N, or at least 2.4N, or at least 3.0N.

The process may be such that the pressure between the first tool's surface and the second tool's (i.e. second) surface, or between the first surface of the first tool and the first sheet (13), is from 20,000 to 80,000 psi.

The tool may comprise studs or teeth placed in rows substantially along the X-direction of the tool and placed in columns along substantially the Y-direction of the tool, and therefore, the composite sheet material and elasticized region thereof may comprises a multitude of troughs along the Y-direction (one or more column of troughs) and along the x-direction (rows of troughs) of said composite sheet.

The process and resulting composite sheet may be such that the troughs (16) of the first sheet (13) have a higher density than the portions of the first sheet (13) not forming said troughs (16), e.g. the portions not contacted by the tool.

Said composite sheet (10) may be a composite barrier sheet, having a hydrostatic head of at least 15 mbar and/ or said first sheet (13) is a barrier sheet having a hydrostatic head of at least 15 mbar.

Said first sheet (13) may be a nonwoven sheet or nonwoven layer, having at least two spunbond webs and at least one meltblown web, said sheet or layer having a basis weight of at least 17 g/ m², and sheet or layer comprising at least 3 g/m² meltblown fibers.

Said composite sheet (10) may comprise, in one embodiment herein, a slit opening (11) and at least two elasticized regions (12 a, b), each of said elasticized regions being positioned along one of the longitudinal sides of said slit opening (11).

In one embodiment herein, in said elastic material (15) may be an elastic band with an average width of 3 mm to 25 mm and whereby the patterned first sheet (13) has a pattern with a width that is from 70% to 300% of the width of the elastic material (15).

As described in detail below, said elasticized region (12) may have a residual strain of less than 20%.

Adhesive may be used to attach the elastic material to the first sheet. In one embodiment, the adhesive is applied as thin filaments, e.g. having an average diameter of less than 200 microns (as measured herein; determined in the composite sheet).

Said elastic material (15) may be a slow-recovery elastomer, as described herein, and then, adhesive may even be omitted, in one embodiment herein.

In one embodiment herein, the composite sheet (10) has an elastic profile of:
1.5Lt by a first load force of less than 1.1N, 3.0Lt by a first load force of less than 2.1N and
4.5Lt by a first load force of less than 3.0N and a second unload force at 4.5Lt of more than 0.9N, a second unload force at 3.0Lt of more than 0.5N and a second unload force at 1.5Lt of more than 0.1N.

### BRIEF DESCRIPTION OF THE FIGURES

Figure 1 shows a perspective view of a preferred absorbent article herein, comprising a composite sheet (10) having patterned, wrinkles elasticized regions (12 a, 12b).
Figure 1a shows a perspective view of a portion A of the article of Figure 1.
Figure 2a shows a cross-sectional view of a fully stretched elasticized region (12) of a composite sheet (10) herein, having a first sheet (13) that is patterned.
Figure 2b shows the elasticized region (12) of Figure 2a in partially stretched state.
Figure 2c shows the elasticized region (12) of Figure 2a in contracted, relaxed state.
Figure 3a shows a cross-sectional view of an alternative elasticized region (12) of a composite sheet (10) herein, in fully stretched state, having a first sheet (13) and a second sheet (14) that are patterned.
Figure 3b shows the elasticized region (12) of Figure 3a in partially stretched state.
Figure 3c shows the elasticized region (12) of Figure 3a in contracted, relaxed state.
Figure 4 shows a perspective view of a first tool and second tool that may be used herein to form the patterned composite sheet (10).
Figure 5 shows a perspective view of an alternative first tool (30) and second tool that may be used herein to form the patterned composite sheet (10).

### DETAILED DESCRIPTION

As used herein, the following terms have the following meanings:
"Absorbent article" means any article that can absorb body fluids and is suitable to be placed in close proximity or against to the skin of a user, e.g. the genitals and/ or anus of the user (including in particular feminine hygiene articles and adult, baby or infant diapers or pads,
including baby, infant, toddler diapers with fasteners, and so-called training or pull-up pants and adult incontinence articles).

As used herein 'front region' and 'back region' refer to the two regions, which are in use, respectively, closest to the front of the wearer and the back of the wearer.

As used herein, the term 'void space' is a cavity in the article present in at least the relaxed state, which serves to accept and contain bodily exudates such as fecal material, for example having a volume of at least 3 or even 5 cm³ in relaxed state.

When used herein, 'longitudinal direction' or "longitudinal dimension" is a direction or dimension, respectively, running "substantially parallel" to the maximum linear direction or dimension of the sheet or article. This is indicated as direction Y, unless stated otherwise.

The "lateral direction", or "lateral dimension", or "transverse direction" or "transverse dimension" is the direction or dimension, respectively, perpendicular to said longitudinal direction or dimension, respectively, and in the same plan of the majority of the article or (composite) sheet and the longitudinal axis. This is indicated as direction X, unless stated otherwise.

"Substantially perpendicular" and "substantially parallel" include directions within 30°, but in preferred embodiments within 20 or 15°, from the exact perpendicular or parallel direction, unless stated or specified otherwise.

"Direction of stretch" when used herein is considered the average direction of stretch.

As used herein, 'along' means 'at least partially (substantially) parallel to and adjacent to'. "Adjacent" includes 'in close proximity with' and 'in contact with'.

As used herein, "opening (11)" (as present in the composite sheet (10) or first sheet (13) or topsheet) means an area circumscribed by said sheet, but where the sheet material is not present, and which is large enough to receive fecal material, for example being at least 2 cm long or wide, or having a surface area of at least 2 cm².

As used herein 'relaxed' or 'relaxed state' or "contracted" or "contracted state" means the state that no forces are applied to the article, to the composite sheet (10), to the elasticized region (12), or to elasticized topsheet or cuff herein (other than naturally occurring forces such as gravity), e.g. when the article is laid on a horizontal surface.

Each embodiment defined by certain properties or dimension for which a value is defined herein is to be understood to include embodiments with functional equivalent properties or dimensions, e.g. a dimension of 0.5 cm has to be understood as meaning "about 0.5 cm".

### Composite sheet (10) and process for making the composite sheet (10)

The composite sheet (10) herein is useful as topsheet or so-called anal cuff for absorbent articles, and it comprises at least one opening to receive fecal material, as described herein in more detail. Said first sheet may comprise said one or more openings in step a) of the process herein.

The composite sheet (10) comprises at least a first sheet (13) and an elastic material (15). Where both the elastic material (15) and the first sheet (13) are present (e.g. one overlaying the other) an elasticized region (12 a and/ or 12 b) is formed. The first sheet (13) may be at least wider, in transverse direction, then the elastic material (15). The first sheet (13) may also be attached to two or more elastic material (15) and their may thus be two or more elasticized regions (12 a, b) in a composite sheet (10).

The composite sheet (10) and said first sheet (13) or part thereof (e.g. all or part of the area of the first sheet (13) forming the elasticized region) is patterned, comprising a multitude of troughs (16). This may be done by use of a patterning tool (30) that pressurizes, and optionally contacts, the first sheet (13) with raised portions (32) extending from the surface (31) of said tool to form said troughs (16) of the first sheet. The first sheet (13), once present in the composite sheet (10), thus comprises typically a multitude of troughs (16) and a multitude of crests (17).

The portions of the first sheet (13) forming said troughs (16) are typically compacted by this patterning step, being thus compacted trough (18) (or compacted attachment areas 18); thus, the portions of the first sheet (13) forming the troughs (16) may thus have a higher density then the portions of the first sheet (13) not forming said trough, e.g. the crests (17), and/ or the portions of the first sheet (13) that may not be patterned.

In one embodiment herein, said first sheet (13) or part thereof is patterned in step c ii)and comprises troughs (16), but said elastic material (15) is not patterned and/ or said elastic material (15) does not comprise troughs (16). In another embodiment, the elastic material (15) may be patterned in step c ii) but this is less than the patterning of the first sheet, i.e. such that the troughs (16) of the elastic material (15) as obtained by the patterning step are smaller in height than the troughs (16) of the first sheet, typically at least 50% less. This may for example be applicable when the elastic material (15) comprises a thermoplastic component and the patterning step involves the application of heat.

In relaxed state, said patterned elasticized region is a wrinkled elasticized region (12), due to the contraction forces of the elastic material (15) that cause the patterned first sheet (13) and patterned elasticized region (12) to wrinkle. Due to the patterning step, the valleys (22) of the wrinkles will coincide with the troughs (16), as also further described herein (and the crests (17) of the first sheet (13) with the peaks (21) of the wrinkles).

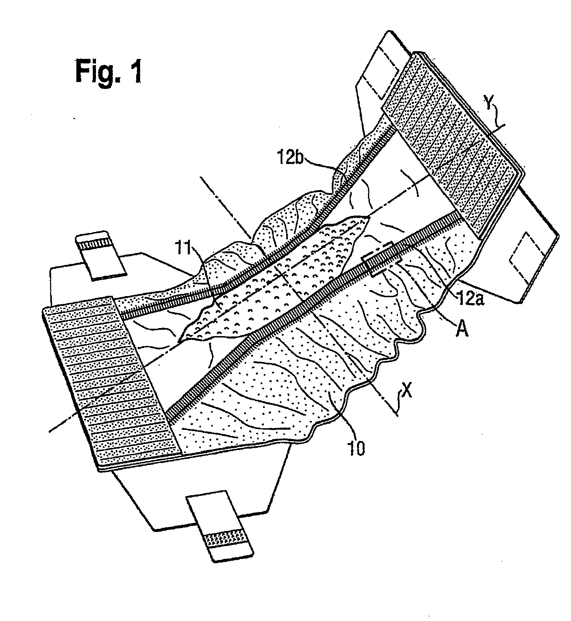

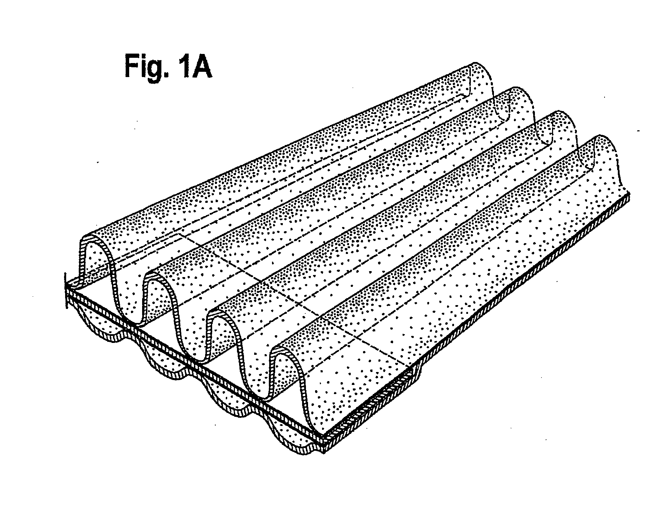

The elastic material (15) is applied to the first sheet (13) in stretched or partially stretched state, preferably the elastic material (15) is at least stretched to 150% or at least 200% of its fully contracted length, when applied to said first sheet; it may be stretched to at least 250% of its original, contracted length, or at least 300% or at least 330%, but for example less than 600%. The elastic material (15) is positioned at least partially, preferably at least 30% or at least 50% of its length, substantially parallel to the longitudinal direction Y. It has an average direction of stretch or elasticity substantially parallel to said longitudinal direction. In one embodiment, the elastic material (15) is applied along a curvilinear pattern, for example such that said elastic material (15) crosses each transverse axis of the composite sheet (10) only once. This is for example shown in Figure 1.

The portion of the first sheet (13) that is submitted to the patterning step, or said first sheet (13) as a whole, is typically not elastic prior to the patterning step and the step applying the elastic material (15).

The patterned first sheet (13) comprises said pattern of troughs (16) over part or all of its length (in longitudinal direction Y) and/ or over all or part of its width (in transverse direction X) of said sheet (13) or of said elasticized region (12) thereof. Thus, the first sheet (13) may also comprise said pattern outside the elasticized region (12).

The first sheet and/ or elasticized region (12 a, b) may comprise at least one, or only one, through in transverse direction X and for example at least 15 troughs, or at least 50 troughs or at least 100 troughs in longitudinal direction, of said region, sheet and/ or article.

At least 10% of the length of the first sheet (13) that forms the elasticized region (12) may comprise said pattern of troughs (16), preferably at least 30% or even at least 40% or even at least 60% or at least 75% or at least 90% or even about 100% of its length.

At least 30% of the width of the part of the first sheet (13) that forms the elasticized region (12) may comprise said pattern of troughs (16), or for example at least 50% or even at least 70% or at least 80% or at least 90% or even about 100%. Furthermore, the width of the part of the first sheet (13) that comprises said troughs (16) may be more than the width of the elasticized region (12) (and more than the width of the elastic material (15)), for example be from 100% to 500% of the average width of the elastic elasticized region (12), or from 100% to 250% or from 100% to 150%. The troughs may be present over substantially the whole width of the first sheet (13).

The number of troughs (16) per cm along the elasticized region (12) substantially in the Y-direction may vary; in one embodiment, the elasticized region (12) and/ or the first sheet (13) has in contracted state an average of from 5 to 25 troughs per cm, or from 5 to 20 troughs per cm, or from 7 to 15 troughs per cm.

The patterning step may be done by applying (indirectly or directly) a patterning surface of a first tool (30) to the surface of the first sheet (13) that does not face the elastic material (15) (but that faces typically in use the user's skin). This tool surface (31) may be a continuous surface, and the tool is for example a patterned roll (30). The first tool's surface (31) comprises raised portions (32).

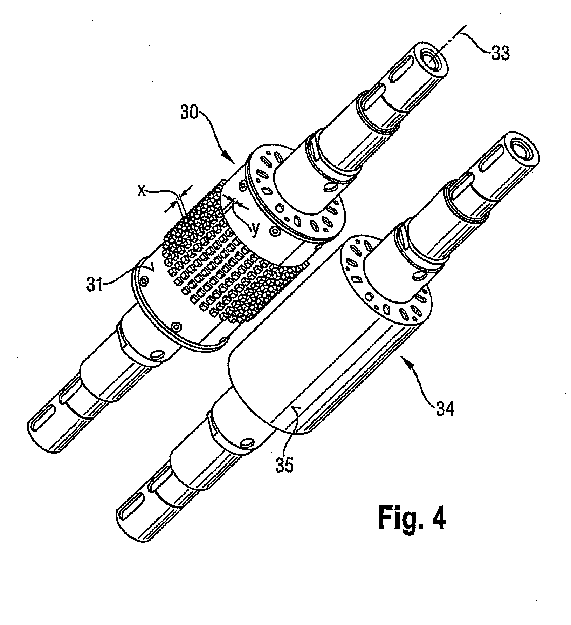

Each raised portion has a width dimension X, substantial parallel to the width of the tool surface, (and typically substantially parallel to the axis (33) of the tool, when the tools is a roll), as shown for example in Figure 4. Each raised portion has also a length dimension Y, substantial parallel to the length of the tool surface, (and typically substantially perpendicular to the axis (33) of the tool, when the tools is a roll), as shown for example in Figure 4.

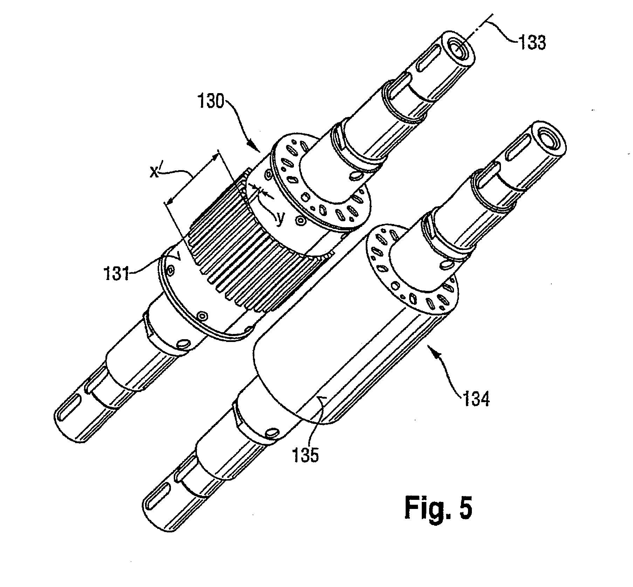

The raised portions (32) may have any shape, for example they may be in the form of studs, or teeth, as for example shown in Figure 4, and/ or ridges, as for example shown in Figure 5. Preferred may be that the surface has a rows of studs and/ or teeth substantially along the x-direction, for example at least 2, or at least 3 or at least 4 of such raised portions per row; whereby the surface also comprises said raised portions in columns in the Y direction (substantially perpendicular to the x-direction), and typically over the length dimension Y of the surface. It may be preferred that the pattern of the raised portions is such that the columns of raised portions in Y-direction are not aligned, so that the pattern is staggering or alternating. The opposite surface of the first sheet (13), which faces the elastic material (15), is pressurized and preferably indirectly contacted by a surface (35) of a second tool (34), to apply a counter pressure to the first tool's surface (31). This second tool's surface (35) is preferably even or non-mating with the first tool's surface (31). Preferred may thus be second tools with an even surface or a surface with non-mating raised portions, but not a surface with mating rasied portions. The second surface (35) may also be a continuous second surface such as a surface of a second roll (34). The second tool's surface (35) may comprise raised portions that contact the raised portions of the first tool (30) in a non-mating manner. In one embodiment, the second tool's surface (35) does not comprise raised portions and it may have an even surface. The second tool (34) may be an anvil roll (34). The elastic material (15), or the second sheet (14) described herein after, may be directly contacted by this second tool (34), such as the anvil roll (34).

A preferred process herein comprises said step c ii) and not step c i), and thus the preferred features herein apply to said step c ii).

The patterning step applies typically a pressure that is large enough to ensure patterning of the first sheet (13) and contacting of the thus formed troughs (16) with the elastic material (15) and possibly aiding attachment thereof to the elastic material (15). The applied pressure may be minimized, to avoid attachment of the crests (17) of the first sheet (13) to the elastic material (15). Suitable pressures may depend on the first sheet's properties, including chemistry of the first sheet, bending rigidity, thickness, and on elastic material (15) properties, including chemistry, thickness; the pressure may also be adjusted depending on whether other attachment means are used, such as heat, or adhesive.

The average pressure applied by the raised portions (32) onto the first sheet (13), or onto the second tool surface (34), may for example be from 10,000 to 100,000 psi, or from 20,000 to 80.000 psi, or for example from 30,000 to 60,000 psi (obtainable by calculation).

Preferred may be that the average distance between the highest point of a raised portions (in x-y plane) of the first tool (30) and highest point of the surface of the second tool (34) is from 0.01 mm to 1.0 mm, or from 0.025 mm to 0.6 mm, or to 0.5mm, or to 0.3mm, or to 0.25 mm.

As mentioned above, the patterning surface of the first tool (30) comprises raised portions that may have any shape. The raised portions may for example be studs, teeth or ridges. Said raised portions have a Z-dimension, e.g. the height of the raised portions, said Z-dimension being perpendicular to the X and Y dimensions of the first tool, e.g. perpendicular to the axis of the tool and out of plane of the tool, when the tool is a roll.

In one preferred embodiment the raised portions (and in particular the ridges and studs herein) include a first flat (distal) surface (in x-y plane), e.g. that in use contacts the sheet material first. This surface may have a Y-dimension (substantially parallel to the MD direction of the sheet and/ or substantially perpendicular to the X-direction of the tool, e.g. the axis of the tool, when the tool is a roll) of for example 0.05 mm to 0.5 mm, or preferably from 0.1 mm or 0.2 mm to 0.4 mm. The X-dimension of such first flat surfaces may be for example from 0.05 to 2.0 mm, or to 1.5 mm; or preferably from 0.1 mm or 0.2 mm to 0.5 mm or to 0.4 mm.

If the raised portion is a ridge, it may have be present over substantially (e.g. 90% or more) of the width (X-direction) of the tool, and its dimension in X-direction may thus be substantially the same as the width of the tool.

Within a column, the Y-direction distance between the highest point of a raised portion, or alternately the centre point of a flat surface of a raised portion, as described above, and the highest point of a neighboring raised portion or the centre point of a flat surface of a neighboring raised portion, in the direction Y may be, in one embodiment herein, from 1.0 mm to 3.0 mm, preferably up to 2.0 mm, or for example from 1.3 mm to 2.0 mm. (This is herein after referred to as the periodicity of the raised portions, in Y-direction.

Within a row, the X-direction distance between the highest point of a raised portion, or alternatively centre point of said surface of a raised portion, as described above, and the highest point of a neighboring raised portion or the centre point of a surface of a neighboring raised portion in the direction X, may be, in one embodiment herein, from 0.2 mm to 2.0mm, preferably up to 1.5 mm, or for example from 0.5 mm to 1.5 mm, or form 0.7mm to 1.3mm.

In a preferred embodiment (in particular when the X-direction of the tool is placed substantially perpendicular to the average direction of stretch of the elastic material, and/ or substantially perpendicular to the Y-direction or machine direction (MD) of the first sheet), the height of a raised portion, as measured from the lowest point of a valley between two neighboring raised portions in the X-direction to the highest point or centre point of the raised portion (herein referred to as X-direction-defined height), is less than the y-direction defined height of the same raised portion, as measured from the foot of a valley between two neighboring raised portions in the Y-direction, to said highest point of said raised portion (herein referred to as y-direction height).

It may be preferred that the average z-direction height of the valleys between raised portions in the Y-direction, or that all Z-direction heights of the valleys between raised portions in the Y-direction, are more than the average Z-direction heights or than all Z-direction heights of the raised portions in the X-direction. It may for example be preferred that the ratio of the Y-direction defined height to the X-direction defined height is at least 3:2, or at least 2:1, or at least 5:2, or at least 3:1, or at least 4:1, or at least 5:1, or for example up to 10:1, or up to 8:1.

The X-direction- defined height may for example be (absolute or on average) from 0.1 mm to 1.0 mm, or for example 0.2 mm to 0.6 mm, or for example to 0.4 mm, or to 0.3 mm. The Y-direction defined height may for example be (on average or absolute) from 0.4 mm to 3 mm, or from 0.6 mm, or from 0.8 mm or from 1.0 mm, to 2.5 mm, or to 2.0 mm.

Thus, in preferred embodiment the composite sheet, or the first sheet, or the wrinkled, patterned elasticized region thereof comprises a pattern of troughs in both the Y-direction and X-direction, whereby said through(s) have an Y-direction height that is more than the X-direction height of said through (said heights being defined as for the raised portions valleys above). It may for example be preferred that the ratio of the Y-direction height to the X-direction height is for example at least 3:2, or at least 2:1 or at least 5:2, or at least 3:1, or at least 4:1, or at least 5:1, or for example up to 10:1, or up to 8:1.

One or more, or all valleys between neighboring raised portions may define a flat surface area (e.g. the lowest surface area of the valley), in substantially the X-Y direction.

In a preferred embodiment, as described above, the tool comprises studs or teeth placed in rows substantially along the X-direction of the tool, and placed in columns along substantially the Y-direction of the tool, and thus, the first sheet, or elasticized regions or combined material of the absorbent article described herein has thus a troughs along the Y-direction and along the X-direction thereof.

In one embodiment, the composite sheet or elasticized region thereof comprises two or three or more elastic strings or strands or bands, aligned along side, each extending at least partially in substantially the Y-direction. Then, it may be preferred that said tool comprises a multitude of teeth or studs in substantially the X-direction and Y-direction of the tool, and said tool is placed in contact or close proximity with the first sheet and in close proximity to the elastic strands such that said valleys between studs or teeth of the tool in the X-direction correspond with the area between said elastic strands or strings in the X-direction, so that thus troughs are formed along the X-direction between said elastic strings or strands.

Thus, a preferred composite sheet herein has a first sheet and an elasticized region with troughs in X and Y direction, and wrinkles in Y-direction, and for example an elasticized region with a multitude of elastic bands, extending at least partially in substantially the Y-direction and providing an elastic force in said substantial Y-direction, whereby said composite sheet comprises one ore more troughs between said elastic strands, strings, or bands, preferably a single trough between two neighboring strands, strings or bands.

In one embodiment herein, the average caliper of the first sheet (13) is less than 50% or less than 40% or less than 25% of the average distance between said raised portions in said Y-direction, as described above.

The patterning tool is directly or indirectly placed onto the first sheet. This may be done such that the width dimension (dimension in direction X) of the tool is within 45°, or within 30° or within 20° or within 10° of the average transverse direction X (width) of the elasticized region (12) and/ or elastic material (15), (said width and transverse direction being perpendicular to the direction of stretch).

In one embodiment, the tool (33) comprises raised portions, being ridges or a rows of teeth or studs, that are each placed within 30°, or within 20° or within 10° or within 10° of the line parallel to the axis of the tool (33); or in one embodiment, between 5° and 20° or 5° and 15°.

It may be preferred that the pattern applied by the patterning tool has thus such a corresponding angle with the elastic material: it may be preferred that for at least 50% of the troughs, or for all of the troughs a line parallel to the width of said troughs has an angle with the line parallel to the direction of stretch of the elastic material at said through or parallel to the MD direction of the first sheet (Y-direction), of from 5°, or from 10° , to 40°, or to 30°, or to 25°, or to 20°.

In one embodiment of the invention, the patterning step serves to also adhere said elastic material to said first sheet (13), optionally in addition to the use of heat bonding or ultrasonic bonding and/ or by use of adhesive. The inventors found that the present process allows attachment and wrinkle formation that is independent of the bonding method used, e.g. independent of the adhesive pattern used. It is believed that the attachment of the first sheet (13) and elastic material (15) is in the process of the invention controlled by the patterning step and pattern thereof, and not by the adhesive pattern (as is typically the case in prior art processes for applying elastic material (15) to nonwovens).

For certain embodiment herein, the inventors even found that the amount of adhesive that may be used, can be significantly reduced without reducing the strength of the bonding of the elastic material to the first sheet: for example less than 20 g/m² (per surface are of said first sheet (13) or of said elastic material), or less than 10 g/m² or less than 8 g/m² of adhesive may be used (e.g. but preferably at least 1 g/m²).

The inventors found that the adhesive (even when used in amount of more than describe above, e.g. more than 20 g/m², as may be desirable in certain embodiments herein) may be applied as very thin filaments or fibers, e.g. the resulting elasticized region, or composite sheet may comprise adhesive filaments of an average or absolute diameter of less than 200 microns, or between 50 and 150 microns, as can be visualized in the elasticized region (12) of the composite sheet (10) by use of microscopy. Adhesives that may be satisfactorily used herein include adhesives manufactured by H. B. Fuller Company of St. Paul, Minnesota and marketed as HL-1620 and HL-1358-XZP and adhesives from National Starch. The adhesive may be applied by known techniques, including spraying, or melt-blowing. The adhesive may be applied in a pattern, such as a spiral or double spiral or omega pattern, or it may in one embodiment be applied randomly. However, it may be applied in an uniform amount per surface area, e.g. per cm². In one embodiment, the adhesive is applied as randomly oriented adhesive fibers in a homogeneous amount, e.g. as mentioned above. In another embodiment, the adhesive may be applied in a pattern of re-occurring shapes, such as a (double) spiral or (double) omega pattern. It may be preferred that the adhesive is applied in a continuous manner, e.g. as a continuous pattern of shapes, whereby said shapes are attached to one another.

It may be preferred, in particular when more than one elastic string or strand is present, or when one or more elastic band is present (per elastic region), such as one or more elastic bands of an average width of 3 mm to 25 mm, that the adhesive is applied in at least two, or at least 3 parallel areas which each extend in the direction of stretch of the elastic material(s), such as for example at least 2, or at least 3 continuous patterns of re-occurring shapes. For example, at least 2, or preferably at least 3, or preferably 4 parallel omega or spiral adhesive patterns may be applied preferably along the average direction of stretch of the elastic material or the Y-direction of the sheet or article, either to the elastic materials or preferably to the sheet.

In one preferred embodiment herein, the adhesive is applied in a pattern of shapes with a certain periodicity of re-occurring patterns, which may be defined by the distance from selected point of a shape to the same selected point on a neighboring shape, such as the distance between the highest point of an omega shape, to the highest point on a neighboring omega shape, along the Y-direction; this distance may for example be from 0.05mm to 2.5 mm, or from 0.05 mm to 2.0 mm or to 1.7 mm. It may for example be useful to have from 5 to 14 omega patterns per cm, or for example from 5 to 12, or from 6 to 12 or to 10 (as can be measured on a said first sheet that is at its full length and width).

It may be preferred that the ratio of the periodicity of the raised portions of the tool and/ or of the periodicity of troughs of the first sheet, to the periodicity of the adhesive pattern is from 0.7, or from 0.8, or from 0.9 to 2.0, or to 1.5, or to 1.3, or it may even be about 1.0 (in substantially the Y-direction).

In one embodiment, use of adhesive may even be omitted, and the patterning step is used to apply pressure and optionally also heat, to achieve the partial attachment of the first sheet's troughs with the elastic material. The resulting composite sheet may thus have elasticized regions that are free of adhesive.

In one embodiment, the patterning step applies heat to the first sheet (13) and/ or to the elastic material (15), and the elastic material (15) and first sheet (13) are attached to one another by said heat and said pressure, (and optionally by adhesive). In this embodiment it may be useful that the elastic material (15) comprises a thermoplastic component that adheres to the first sheet (13) under the process temperature of this step. The elastic material (15) may have an elastic component that is thermoplastic and adheres to the first sheet (13) under the process heat of the patterning step, and/ or it may comprise an elastic component and a thermoplastic component, for example a thermoplastic coating on a elastomeric material, that adheres to the first sheet (13) under process temperature of the patterning step. Process temperatures may for example be between 30°C and 165°C, or between 40°C and 150°C or 50°C and 150°C.

Preferred thermoplastic components are described herein below.

The process herein may also be done under cooling of the first sheet (13) and/ or composite sheet (10) in step c), d) and/ or e), e.g. under cooling of the patterning tool's surface, for example by cooling of the first tool's surface (31) with raised portions and/ or by cooling of the second tools' surface. For example, the process may be done such that the first sheet (13) and/ or the resulting composite sheet (10) is contacted by a surface that has a temperature of between -20°C and 15°C or between -20°C and 10°C or between -10°C and 5°C. A preferred equipment herein is a combination of a first tool with a patterning surface with raised portions and an opposing second tool, whereby said first and/ or second tool comprises a cooling system, said tools being as described herein, e.g. having the raised portions as described above, including the preferred raised portion dimensions and X-direction valley dimensions and/ or Y direction valley dimensions.

The composite sheet (10) herein comprises, in relaxed state, wrinkles, whereof the peaks (21) are formed by the first sheet (13) and the valleys (22) are formed by the first sheet's troughs (16) and elastic material (15).

The composite sheet (10) may have an elasticized region (12) with a first sheet (13) with wrinkles that are uniform, e.g. a uniform wrinkle pattern. The first sheet (13) may have wrinkles that have a uniform wrinkle height and/ or uniform wrinkle density, namely:
a) at a partial elongation of the composite sheet (10) of ε, which is 2/3 (i.e. 66.7 %) of its fully stretched length, said first sheet (13) present in said elasticized region (12) may have wrinkles with an average wrinkle height H_{w} (as measured by the "Primos" method set out below, using PRIMOS equipment) of from 150 microns to 600 microns, or from at least 180 microns or at least 200 microns and/ or up to 550 microns , or up to 500 microns. This height is the distance between the highest point of a peak of a wrinkle to the lowest point of the valley of the wrinkle of the first sheet, as described in the "Method" section.
   The average wrinkle height above may have a standard deviation (STD) of less than 100 microns, preferably less than 75 microns, or less than 50 microns and the RSD (being the STD/ H_{w}) is less than 30% or less than 20% or less than 10%.
b) In addition, or alternatively, at said elongation ε of 2/3 (=66.7%) of the fully stretched length (as set out above, and measured with the Primos method) the first sheet (13) of the elasticized region (12) of the composite sheet (10) herein may have wrinkles such that the average distance between the highest points of neighboring peaks, or between centre point the highest regions of neighboring peaks (what ever is applicable) of the wrinkles is from 500 to 1500 microns, or from 750 to 1400 microns, or from 800 to 1300 microns or from 900 to 1200 microns, whereby the standard deviation may be less than 250 microns, or less than 200 microns or less than 100 microns.
   The RSD (=STD/ average) may hereby be less than 30%, or less than 20% or less than 10%.
c) In addition, or alternatively to b) above, at said elongation ε of 2/3 (=66.7%) of the fully stretched length (as set out above, and measured with the Primos method) the first sheet (13) of the elasticized region (12) of the composite sheet (10) herein may have wrinkles such that the average distance between the lowest points of neighboring valleys or between centre point the lowest regions of neighboring valleys (what ever is applicable) of the wrinkles is from 500 to 1500 microns, or from 750 to 1400 microns, or from 800 to 1300 microns or from 900 to 1200 microns, whereby the standard deviation may be less than 250 microns, or less than 200 microns or less than 100 microns.
   The RSD (=STD/ average) may hereby be less than 30%, or less than 20% or less than 10%.
c) Alternatively, or in addition, at least 80% or at least 90% or even 100% of the wrinkles of the first sheet (13) (as defined above and measured above) have a wrinkle height between 650 microns and 180 microns, or between 600 microns and 180 or 200 microns, or between 500 microns and 180 or 200 or 300 microns; for example, less than 10% or even less than 5% of the wrinkles has a height of 700 or more.
d) Alternatively, or in addition, at said elongation ε of 2/3(=66.7%) of the fully stretched length, the first sheet (13) of the elasticized region (12) herein may have an average wrinkle density of from 5 to 25 wrinkles per cm or from 6 to 20, from 6 to 15 per cm or from 6 to 10 per cm, or from 6 to 9 per cm, as measured with the Primos method and as set out herein, and having a RSD of less than 30% or less than 20% or less than 10%.

In one embodiment, the first sheet (13) is folded around the elastic material (15) as a C-fold, and it thus serves also as covering sheet material, or herein referred to "second sheet (14)", for the opposite side of the elastic material (15).

Alternatively, or in addition, the opposite surface of the elastic material (15), not facing the first sheet (13) may be contacted and attached to an additional covering sheet material, i.e. second sheet (14).

In one embodiment herein, the first sheet (13) and elastic material (15) are positioned adjacent a second sheet (14) in any of the process steps b) c) or d); the elastic material may then be present between said first and second sheet. The second sheet (15) may be present during the patterning step.

This second sheet may thus be formed from the first sheet, or it may be an additional sheet, made of any material that is pliable and can form wrinkles under the elastic forces of the elastic material. Preferred are nonwovens as described herein, or in one embodiment, it may be a nonwoven as described hereinafter as preferred nonwovens for the first sheet (13). The second sheet (14) is typically attached to the elastic material (15) in stretched state, by any method, including those described herein for the first sheet. The second sheet (14) may be patterned with troughs (16) and attached to the elastic material (15) with said troughs (16), by the process described herein for the first sheet (13). This may be done in a separate process step prior to or after the patterning process step of the first sheet (13), as described herein, or it may be done at the same time as the pattering step to pattern the first sheet. This may be done by use of the first tool (30) too, as described herein, or it may be done with a second tool (34) having raised portions whereof the top surface contacts the top surface of the raised portions (31) of the first tool. In one embodiment, the second sheet (14) is combined with the elastic material (15) and the first sheet material (13) in step c) or d) to form a combined material that is then pressurized by said first tool (30), as described herein, to form a pattern of troughs (16) in both the first and second sheet, said troughs having for example the wrinkle uniformity as described herein. Either the second or the first sheet may be contacted by said first tool.

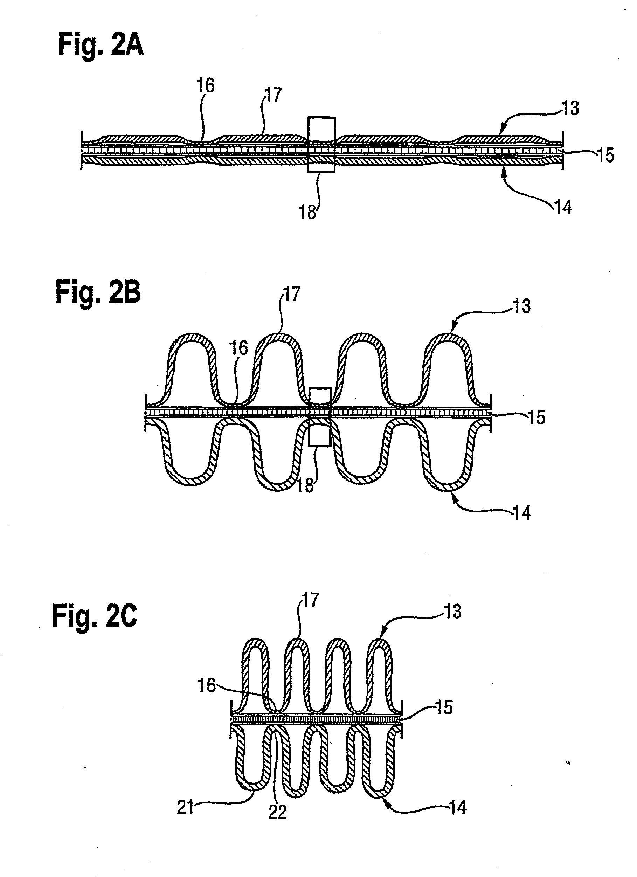

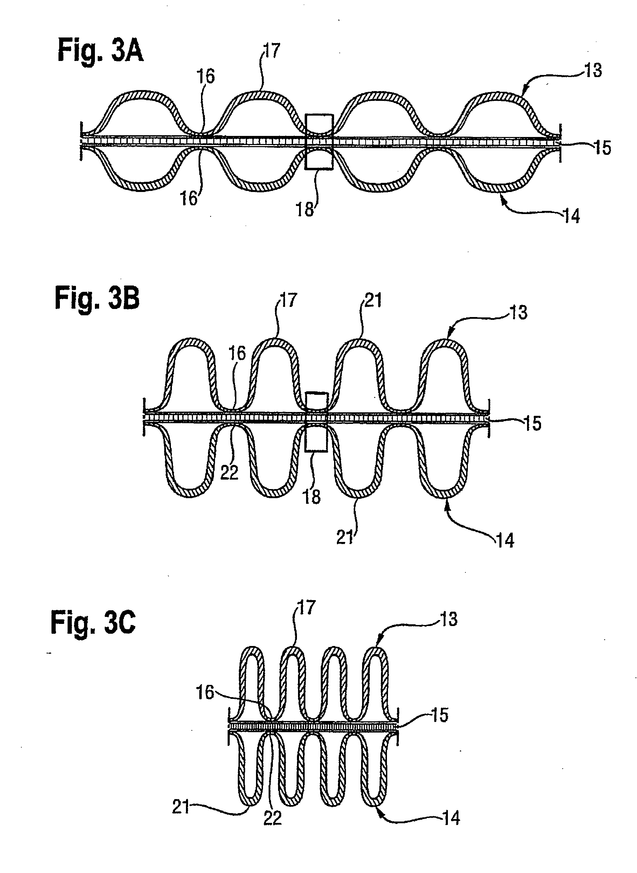

An embodiment whereby both sides of the elastic material (15) of the elasticized region (12) are contacted and adhered to, respectively a first sheet (13) and a second sheet (14) is shown in Figures 2a, b, c and 3 a, b, c. As shown in Figure 3a, b, c, the second sheet (14) may also be patterned as described herein, and this may or may not be the same pattern of troughs as the pattern of troughs of the first sheet (13). It may thus also comprise troughs (16) that are compacted, having a higher density than the portions of the second sheet (14) that not form the troughs of the second sheet (14), just as described herein for the first sheet (13). It may have the uniform wrinkle pattern as set out above.

Alternatively, as shown in Figure 2a, b, c, the second sheet (14) may be not patterned with troughs (16) and is may have a non-uniform wrinkle pattern, not fulfilling the requirements as set out herein for the first sheet.

The composite sheet (10) that comprises a wrinkled and patterned elasticized region (12) as described herein, having a first sheet (13) and any type of second sheet (14) may have a peel force of at least 1.4 N, preferably at least 2.0N or at least 2.4N, or at least 3.0N.

The elasticized region (12) herein may have a residual strain of less than 30%, or less than 20%, or between 1% and 30% or between 5 % and 20%, as measured by the method described herein below.

### Elastic material (15)

The elastic material (15) herein may be any elastic material (15) and it may be in any form or shape. The elastic material (15) may be in the form of a string, having a thickness to width ratio of 1:1 to 1: 4, for example having a substantially circular cross section, or it may be in the form of a band, having a thickness to width ratio of more than 1:4. The elasticized region (12a, b) herein may comprise a multitude of strings or bands of elastic material.

The elastic materials (15) used herein may be very thin, for example having a thickness or caliper (e.g. gauge) of up to about 200 microns, or even up to 150 microns or even up to 110 microns, or up to 100 microns. The elastic material herein may have any minimum caliper, but it may be at least 20 microns, more preferably at least 40 microns, or even at least 60 microns, as defined herein. The elastic material (15) may have a thickness of about 70 to 100 microns.

Suitable elastic materials may be such that they provide the following elastic profile to the composite sheet (10):
1.5Lt by a first load force of less than 1.1N, 3.0Lt by a first load force of less than 2.1N and 4.5Lt by a first load force of less than 3.0N and a second unload force at 4.5Lt of more than 0.9N, a second unload force at 3.0Lt of more than 0.5N and a second unload force at 1.5Lt of more than 0.1N.

It may have an elastic profile of:
1.5Lt by a first load force of less than 1.1N, 3.0Lt by a first load force of less than 2.1N and 4.5Lt by a first load force of less than 3.0N and a second unload force at 4.5Lt of more than 0.9N, a second unload force at 3.0Lt of more than 0.5 and a second unload force at 1.5Lt of more than 0.1N.

The elastic profile of the composite sheet (10) herein may be:
1.5Lt by a first load force of less than 0.6 N, 3.0Lt by a first load force of less than 1.1N and 4.5Lt by a first load force of less than 1.5N and a second unload force at 4.5Lt of more than 0.9N, a second unload force at 3.0Lt of more than 0.5N and a second unload force at 1.5Lt of more than 0.1N.

Hereby, Lt is the length of the composite sheet (10) or, (if the composite sheet (10) is a topsheet of the article, Lt is the shortened topsheet length), as can be determined as set out in EP1201212-A (referred to as shortened topsheet length). This patent also describes the test method to obtain the above and below elastic profile.

Suitable elastic materials include cross-linked elastic polymers, including cross-linked rubbers. A suitable elastic material (15) is for example 2L-89, available from Fulflex, (Limerick, Ireland).

The elastic material useful in the absorbent articles herein may be a so-called slow recovery elastomer, as described in co-pending application WO2005/020222 (EP application number 056760373.0). When used herein, the slow-recovery elastic is typically an elastomer which exhibits a normalized unload force at 37°C of at least about 0.04 N/mm² as measured by the Two Cycle Hysteresis Test set out in said patent application. The slow recovery elastomer exhibits at least a 20% or at least 35% or at least 50% post elongation strain at 22°C after 15 seconds of recovery, as measured by the Post Elongation Recovery Test set out in the above application. Said slow recovery elastomer may comprise about 20% to about 70%, by weight, of at least one elastomeric polymer; and the remaining portion being components, such as those described below.

It may have a normalized unload force at 37°C of at least about 0.16 N/mm² and at least a 10% opost elongation strain at 22°C after 15 seconds of recovery.

The slow recovery elastomer may exhibit a normalized unload force at 37°C of greater than about 0.04 N/mm² and a post elongation strain of at least about 20% after 15 seconds of recovery at 22°C, as described in said co-pending application.

Preferably, the slow recovery elastomer exhibits a normalized unload force of greater than about 0.08 N/mm² at 37°C, and, most preferably, exhibits a normalized unload force of greater than about 0.12 N/mm² at 37°C. In other suitable embodiments, at 22°C the slow recovery elastomer exhibits a post elongation strain from about 75% to about 150% after 15 seconds of recovery. However, post elongation strain after 15 seconds of recovery may exceed about 170% at 22°C. Furthermore, the slow recovery elastomers may exhibit a specified post elongation strain at 22°C after 30 seconds, 60 seconds, or three minutes of recovery. In certain embodiments, the slow recovery elastomer may exhibit at least about a 70% post elongation strain after 30 seconds of recovery at 22°C. In other embodiments, the slow recovery elastomer may exhibit at least about a 40% post elongation strain after 60 seconds of recovery at 22°C.

Suitable elastomeric polymers comprise styrenic block copolymers, natural and synthetic rubbers, polyisoprene, neoprene, polyurethanes, silicone rubbers, hydrocarbon elastomers, ionomers, and the like. In one embodiment, the elastomeric polymer may be a block copolymer. A number of block copolymers may be used to prepare the slow recovery elastomer including multi-block, tapered block and star block copolymers. Generally, the block copolymers suitable for use in the slow recovery elastomer may exhibit both elastomeric and thermoplastic characteristics. In such block copolymers a hard block (or segment) may have a glass transition temperature (Tg) greater than about 25 °C or is crystalline or semicrystalline with a melting temperature (Tm) above about 25°C. Preferably, the hard block has a Tg greater than about 35 °C or is crystalline or semicrystalline with a Tm above about 35 °C. The hard block portion is typically derived from vinyl monomers including vinyl arenes such as styrene and alpha-methyl-styrene or combinations thereof.

Glass transition temperatures referred to herein are determined by tensile dynamic mechanical analysis performed in the linear elasticized region (12) of the material at a frequency of 1 Hz using a temperature ramp method. Suitably, film samples with a uniform thickness of about 0.3 mm or less may be used with a temperature ramp rate of about 1°C/min or slower. The Tan δ peak temperature is taken as the Tg of the particular material or phase.

Crystalline melting temperatures referred to herein are determined by Differential Scanning Calorimetry using a temperature ramp rate of 10°C/min. The melting endotherm peak temperature is taken as the Tm of the particular crystalline region.

The block copolymers may comprise a soft block (or segment). The soft block generally exhibits a sufficiently low glass transition temperature and/or melting temperature so as not to form glassy or crystalline regions at the use temperature of the copolymer. In one embodiment, the use temperature may be between about room temperature (about 22°C) and about body temperature (about 32°C). However, other use temperatures are feasible and within the scope of this invention. Such soft blocks are generally physically incompatible with the hard blocks and form separate regions, domains, or phases.

The soft block portion may be a polymer derived from conjugated aliphatic diene monomers. Typically, the soft block monomers contain fewer than about 6 carbon atoms. Suitable diene monomers include butadiene, isoprene, and the like. Particularly preferred soft block polymers include poly(butadiene) and poly(isoprene). Furthermore, it is envisioned that the soft block may be modified to tailor the Tg of the soft block. For example, a random copolymer of isoprene and styrene or a graft of styrene onto poly(isoprene) may be used. In such cases, lower amounts of the modifying resin may be used.

Suitable block copolymers for use in this invention may comprise at least one hard block (A) and at least one soft block (B). In a preferred embodiment, the block copolymer may be an A-B-A triblock copolymer, an A-B-A-B tetrablock copolymer, or an A-B-A-B-A pentablock copolymer. Also, useful herein are triblock copolymers having endblocks A and A', wherein A and A' may be derived from different vinyl compounds.

Preferred elastomeric polymers include styrene-olefin-styrene triblock copolymers such as styrene-butadiene-styrene (S-B-S), styrene-ethylene/butylene-styrene (S-EB-S), styrene-ethylene/propylene-styrene (S-EP-S), styrene-isoprene-styrene (S-I-S), hydrogenated polystyrene-isoprene/butadiene-styrene (S-EEP-S), and mixtures thereof. The block copolymers may be employed alone or in a blend of block copolymers. Particularly preferred block copolymers include styrene-butadiene-styrene (S-B-S) and styrene-isoprene-styrene (S-I-S) block copolymers. Such linear block copolymers of styrene-butadiene-styrene (S-B-S) and styrene-isoprene-styrene (S-I-S) are commercially available under the trade designation Vector from Dexco Polymers L.P., Houston, TX, and under the trade designation Kraton from Kraton Polymers, Houston, TX.

Modifying resins may be used; they should have a sufficiently high average molecular weight such that the glass transition temperature of the soft block is increased resulting in an increase of post elongation strain at 22°C after 15 seconds of recovery. The slow recovery elastomer may comprise the modifying resin in amounts from about 0% to about 60% by weight. Preferably, the composition comprises from about 20% to about 55% and even more preferably from about 35% to about 45% of the modifying resin.

Suitable modifying resins useful herein may have glass transition temperatures ranging from about 60°C to about 180°C, more preferably from about 70°C to about 150°C, and more preferably from about 90°C to about 130°C.

Modifying resins useful herein include, unhydrogenated C5 hydrocarbon resins or C9 hydrocarbon resins, partially and fully hydrogenated C5 hydrocarbon resins or C9 hydrocarbon resins; cycloaliphatic resins; terpene resins; polystyrene and styrene oligomers; poly(t-butylstyrene) or oligomers thereof; rosin and rosin derivatives; coumarone indenes; polycyclopentadiene and oligomers thereof; polymethylstyrene or oligomers thereof; phenolic resins; indene polymers, oligomers and copolymers; acrylate and methacrylate oligomers, polymers, or copolymers; derivatives thereof; and combinations thereof. Preferably, the resin is selected from the group consisting of the oligomers, polymers and/or copolymers derived from: t-butylstyrene, cyclopentadiene, iso-bornyl methacrylate, methyl methacrylate, isobutyl methacrylate, indene, coumarone, vinylcyclohexane, methylstyrene, and 3,3,5-trimethylcyclohexyl methacrylate. Preferred modifying resins also include alicyclic terpenes, hydrocarbon resins, cycloaliphatic resins, poly-beta-pinene, terpene phenolic resins, and combinations thereof. "C5 hydrocarbon resins" and "C9 hydrocarbon resins" are disclosed in U.S. Patent No. 6,310,154.

The slow recovery elastomer may exhibit temperature responsiveness. In one embodiment, a temperature responsive slow recovery elastomer may exhibit a post elongation strain after 15 seconds at 32°C that is at least 35% less than the post elongation strain after 15 seconds at 22°C. Preferably, at least a 50% reduction in post elongation strain is exhibited. Most preferably, at least a 75% reduction in post elongation strain is exhibited. It is believed that a slow recovery elastomer exhibiting temperature responsiveness may further facilitate diaper application. When the absorbent article is applied at about room temperature (*e.g*., approximately 22°C), the slow recovery elastomer exhibits a relatively high degree of post elongation strain for a prescribed period of time. Upon application of the diaper, the slow recovery elastomer will rise in temperature because of the close proximity of the wearer's skin. As the temperature of the slow recovery elastomer increases and nears body temperature (*e.g*., approximately 32°C), the reduced post elongation strain is exhibited. Temperature responsiveness allows for application of the diaper without "snap-back" while providing for increased recovery after application.

Other components may be stabilizers, antioxidants, and bacteriostats (to avoid degradation of the slow recovery elastomer). Generally, the additive or additives may account for about 0.01% to about 60% or to about 25% or to about 10% of the total weight of the slow recovery elastomer composition.

Other optional additives include thermoplastic polymers or thermoplastic polymer compositions which preferentially associate with the hard blocks or segments of the block copolymers. Without intending to be bound by theory, it is believed that these thermoplastic polymers become incorporated into the entangled three-dimensional network structure of the hard phase. This entangled network structure can provide improved tensile, elastic and stress relaxation properties of the elastomeric composition. Where the elastomeric polymer comprises a styrenic block copolymer, thermoplastic polymer additives such as polyphenylene oxide and vinylarene polymers derived from monomers including styrene, alpha-methyl styrene, para-methyl styrene, other alkyl styrene derivatives, vinyl toluene, and mixtures thereof, are useful in the present invention because they are generally considered to be chemically compatible with the styrenic hard blocks of the block copolymer.

Processing aids may alos be included, such as processing oils, which are well known in the art and include synthetic and natural oils, naphthenic oils, paraffinic oils, olefin oligomers and low molecular weight polymers, vegetable oils, animal oils, and derivatives of such including hydrogenated versions, preferably a mineral oil. Viscosity modifiers may also be used, such as those well known in the art. For example, petroleum derived waxes can be used to reduce the viscosity of the slow recovery elastomer in thermal processing. Suitable waxes include low number-average molecular weight (*e.g*., 600-6000) polyethylene; petroleum waxes such as paraffin wax and microcrystalline wax; atactic polypropylene; synthetic waxes made by polymerizing carbon monoxide and hydrogen such as Fischer-Tropsch wax; and polyolefin waxes.

### First sheet (13)

The first sheet (13) may be any sheet material useful for absorbent articles, including woven sheets, nonwoven sheet, films.

In a preferred embodiment, the first sheet (13) is not elastically extendable or stretchable, e.g. under normal process strain.

In one preferred embodiment herein the first sheet (13) is a nonwoven sheet. The first sheet (13) may be a nonwoven sheet that is laminates of two or more nonwoven layers and/ or two or more nonwoven webs.

As used herein, a "nonwoven web" is a single web, whilst a "nonwoven layer" may comprise a multitude of nonwoven webs; a "nonwoven sheet" may comprise a multitude of nonwoven layers.

The first sheet (13) may be a (nonwoven) barrier sheet, such as first sheets with a hydrostatic head value (measured with the hydrostatic head test set out herein) of at least 10 mbar, or at least 15 mbar, or at least 18 mbar, or in one embodiment, at least 20 mbar, or at least 25 mbar, or at least 28 mbar, or at least 30 mbar, or in one embodiment at least 35 mbar.

In one embodiment, the hydrostatic head of the first sheet (13) is between 10 and 50 mbar.

In one embodiment, the composite sheet (10) may alternatively or in addition have the hydrostatic head values above.

Said first sheet (13) or composite sheet (10) is considered to have the above hydrostatic head values if it has this value at any part of the first sheet (13) material, excluding elasticized areas or areas with edges that are attached to other materials, prior to attachment to the elastic material (15) in the process herein; and/ or if it has this value at any part of the first sheet (13) after attachment to the elastic material (15), excluding at the elasticized region (12) or at areas that have edges that are attached to other materials; i.e. the measurement is done on a sample of the sheet that does not comprise elastic material (15) or edges of the first sheet (13) that area attached to another material. In one embodiment, the first sheet (13) and/ or composite sheet has a surface area free of elastics or edges of at least 2.5 cm x 2.5 cm.

The first sheet (13) may have a bending rigidity of 20 grams or less, or 16 grams or less, or even 14 grams or less or 12 grams or less, as measured with the handle-o-meter test set out herein. Alternatively, or in addition the composite sheet (10) may have a bending rigidity of less than 35 grams, or less than 30 grams or less than 25 grams or less than 20 grams or less than 18 grams, or as described above.

A first sheet (13) or composite sheet (10) herein is considered to have the above bending rigidity values if it has this value at any part of the material, excluding areas comprising elastic material, including the elasticized region (12) herein, or edges attached to other materials (these areas should not be included in the test).

The bending rigidity as referred to herein, and measured with the method herein, is the rigidity of said sheet in any direction, unless specified otherwise.

In one embodiment, the first sheet (13) comprises at one surface, e.g. that is not to be attached to the elastic material (15) and that faces the patterning tool, a nonwoven web comprising fibers with an average fiber direction, and said first sheet (13) has a bending rigidity of the values specified above, in said fiber direction. Preferred nonwoven webs that contact the patterning tool and/ or that are on the surface of the first sheet (13) that is not attached to the elastic material (15), are spunbond webs (with fibers with an average fiber direction).

The average fiber direction may typically be the longitudinal direction of the composite sheet (10) and/ or the machine direction (MD) of the absorbent article.

The first sheet (13) or the composite sheet (10) has in one embodiment a low surface tension strike through value, as determined by the method described herein, of at least 30 seconds, or for example at least 50 seconds, or even at least 60 seconds. The strike through value may be less than 200 seconds, or less than 150 seconds or less than 100 seconds. A first sheet (13) or composite sheet (10) is considered to have the above low surface tension strike through values if it has this value at any part of the material, excluding areas comprising elastic material (15), including the elasticized region, or edges being attached to other materials.

As mentioned above, in one embodiment the first sheet (13) is a nonwoven sheet that comprises two or more nonwoven layers that are attached to one another, but in one embodiment, not fully (i.e. not 100%) laminated to one another. In one embodiment, said two (or more) nonwoven layers have an attachment area of 60% or less, or 40% or less or even 20% or less (of the total area of overlap between two neighboring nonwoven layers). In one embodiment, the first sheet (13) comprises two or more nonwoven layers that are attached to one another along the side edges of the overlap area, e.g. along the edges of each or one of the nonwoven webs (periphery) and optionally the area where elastic material (15) is present, and the nonwoven layer comprises areas, e.g. of at least 0.5 cm², where both layers are present but not attached to one another. In one embodiment, the first sheet (13) is such that at least two nonwoven layers thereof are only partially attached to one another and there is at least one area of 2.5 x 2.5 cm that is not attached (and does not comprise elastics or edges).

In one embodiment, the first sheet (13) is a nonwoven sheet that comprises nano-fibers, which have an average diameter of 1.0 microns or less. Preferred may be that the first sheet (13) comprises two or more nonwoven layers, whereof one or more, or each, comprise a nonwoven web that comprises such nano-fibers. The nonwoven sheet or layer or web may for example comprise at least 2 g/m² of nano-fibers, or at least 3 g/m² or at least 5 g/m² of nano-fibers. The nano-fibers may have an average diameter of 0.8 microns or less, or 0.6 microns or less. The nano-fibers may be made by known melt fibrillation methods or melt film fibrillation methods, such as described in US6,315,806 and US6,695,992. Preferred nano-fiber webs and layers are described in co-pending application WO2005/103355.

In one embodiment, the first sheet (13) is a nonwoven sheet that has at least one nonwoven layer, comprising at least one nonwoven web of meltblown fibers, for example present at a weight level of at least 5 g/ m² by weight of the nonwoven layer, or for example at least 5.7 g/m², or at least 7 g/ m², but for example less than 20 or less than 15 g/m² by weight of the nonwoven layer. The basis weight of the first sheet (13) is generally at least 5 g/m², or at least 7 g/m², or at least 10 g/m², or at least 17 g/m², or at least 22 g/m² ; it may be preferred that the basis weight is 60 g/ m² or less, or 45 g/m² or less or 40 g/m² or less or 35 g/m² or less.

If the first sheet (13) comprises two nonwoven layers, each comprising two or more nonwoven webs, it may be preferred that the basis weight of each of the nonwoven layers present in said first sheet (13) is 24 g/m² or less, or 22 g/m² or less or 18 g/m² or less, and/ or at least 5g/m² or at least 7 g/m² or at least 10 g/m².

Suitable first sheets herein are: a nonwoven sheet comprising a 17 or 22 gsm (g/m²) SMMMS or SMMS nonwoven layer attached to (but not laminated to) another 17 or 22 gsm SMMMS or SMMS nonwoven layer (whereof for example the meltblown level of each layer is 5.7 or 7.3 gsm respectively), including for example a nonwoven sheet comprising 22 gsm SMMMS nonwoven layer, with for example 7.3gsm meltblown fibers, attached to 17 gsm SMMMS or SMMS nonwoven layer, comprising for example 5.7 gsm meltblown fibers; a nonwoven sheet comprising a 17 gsm or 22 gsm SMS or SNS nonwoven layer, attached to another 17 gsm or 22 gsm SNS or SMS nonwoven layer.

The first sheet (13) may comprise a hydrophobic surface coating, such as known in the art, for example a wax, or preferably a hydrophobic surface coating comprising one or more silicone polymers or fluorinated polymers. Suitable silicone polymers are for example selected from the group consisting of silicone MQ resins, polydimethysiloxanes, crosslinked silicones, silicone liquid elastomers, and combinations thereof. Typically, the molecular weight of such silicone polymers should be at least about 4000 MW, preferably at least about 10,000 MW, more preferably at least about 15,000 MW, even more preferably at least about 20,000 MW, and most preferably at least about 25,000 MW. Preferred polydimethylsiloxanes are selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof. Suitable fluorinated polymers are selected from the group consisting of telomers and polymers containing tetrafluoroethylene and/or perfluorinated alkyl chains. For instance, fluorinated surfactants, which are commercially available from Dupont under the tradename Zonyl®, are suitable for use herein. In particular, Zonyl® 321, 329, 8740, 9027, and 9360 are well suited for use in the present invention. Additionally, other Zonyl® materials include fluroadditives like micro-powders may be useful herein. These include, but are not limited to Zonyl® MP1100, MP1200, MP1400, MP1500J, MP1600N, TE-3667N (which is a water dispersion). Preferably, the coating is free of aminosilicones.

These materials may be deposited onto the composite sheet (10) in amounts of from at least about 0.01 gsm (gram of material/square meter of composite sheet), or from at least about 0.05 gsm, and or from at least about 0.1gsm.

Preferred first sheets or composite sheets (10) herein are considered urine-impermeable and feces impermeable and thus suitable herein, when they have a low surface energy and a uniform pore size distribution, for example with the low surface energy values, pore sizes and air permeability values described in co-pending application EP-A-1417945. For example, useful may be materials that are substantially impermeable materials with an alcohol repellency of at least 5 or at least 6 or at least 7, or at least 8; having for example a surface energy of between 20 and 35 mN/m; optionally having a contact angle with water of above 100°; and optionally having a mean pore size of less than 50 microns, preferably less than 30 microns, or less than 20 microns, but optionally at least 2 microns, or at least 5 microns. The first sheet (13) or composite sheet (10) may have an air permeability of at least 3 Darcy, or at least 10 Darcy, or at least 20 Darcy, or at least 30 Darcy.

### Absorbent articles

The absorbent article herein is an absorbent article to be worn in close proximity to (or in contact with) at least the anus of a wearer, such as adult incontinence products, such as briefs, pads or diapers, and baby diapers, toddler diapers, e.g. diapers with fasteners and pull-on diapers (or pants).

The composite sheet (10) herein is or forms part of a topsheet of said article, that faces the wearer in use, e.g. such that the composite sheet (10) may contact the skin of the user. Thus, the topsheet comprises the composite sheet (10) or is formed by the composite sheet (10) or it may consist of the composite sheet (10).

The composite sheet or topsheet, and optionally the first sheet, has one or more (preferably one or two, most preferably one) openings (11) to receive fecal material.

An exemplary diaper with such a composite sheet/ topsheet is shown in Figure 1.

Preferably, the opening is in the form of a single slit opening (11). The opening is preferably present in part of the front region of said topsheet (from the transverse axis X to the front of the diaper, and thus in use towards the front of the user) and in part of the back region of the topsheet. Preferably, the topsheet has a slit opening (11), which has a longitudinal dimension (length; Y-direction) substantially parallel to the longitudinal axis Y of the topsheet and of the article. The direction of stretch of the composite sheet (10) is preferably along said longitudinal axis. It may be that (in stretched state) the opening(s) (11) of the topsheet is (are) configured such that from 20% to 40%, or from 20% to 30% of the length of the opening (or total length of the openings) extends from the transverse axis of the topsheet towards the front edge of the topsheet, and the remaining percentage extends towards the back edge of the topsheet.

The dimensions and exact shape of the opening(s) (11) may vary, depending on the size of the topsheet and/ or the absorbent article. For example, in a preferred embodiment the opening is in the form of a slit opening with substantially parallel longitudinal side edges, which are connected in the front and back by V-shaped or rounded V-shaped front and back edges, wherein both the front and back V-shaped edges comprise two angled edges. The maximum length of the slit opening (11) (in fully stretched state) may be for example 40% to 90% or 50% to 80%, or about 60% to 70%, of the total length L of the absorbent article. The average width of the opening (11) herein, in 66.7% stretched state, is preferably from 5% to 30%, or from 10% to 25%, of the average width of the topsheet (including opening width), or from a size 4 diaper, 15 mm to 60 mm, or from 20 mm to 40mm.

The topsheet, being the composite sheet (10) or comprising the composite, has said elasticized region (e.g. 12 a and/ or b) of the composite sheet (10) such that it extend preferably along said longitudinal side edge of the opening(s) towards or completely to the front and/ or back transverse edge of the topsheet. The elasticized regions (12a, 12b) are preferably longer than the opening or openings (11). Said topsheet comprising one or more openings (11), and being formed by said composite sheet (10) or comprising said composite sheet (10), preferably comprises at least two elasticized regions (12 a, b) as described herein, each along either longitudinally extending side edge of the opening or openings (11). Hereby said elasticized regions (12a, b), and preferably said side edges, are typically mirror images of one another in the y-axis of the topsheet or article.

The width of the elasticized regions (12a, 12b) will vary, typically depending on the exact dimensions of the topsheet or cuff and/ or the article, for example the elasticized region (12) may have an average width of about 1 mm to 40 mm, or 2 mm to 30 mm, or 2 mm, or even 3 mm to 20 mm, or 5 to 12 mm (in relaxed, contracted state).

The front end portions of two opposing elasticized regions (12a, 12b) may bend away from one another (in the plane of the topsheet), so that the distance between the end edges of the opposing front end portions of two opposing elasticized regions (12a, 12b) is larger that the distance between the two longitudinal centre points of two opposing elasticized regions (12a, 12b), and equally, the distance between the end edges of the opposing back end portions of two opposing elasticized regions (12a, 12b) may be larger that the distance between the longitudinal centre points of two opposing elasticized regions. This is further exemplified in Figure 1.

The front end portion of an elasticized region (12) may have an angle with a longitudinal line through the centre point of the elasticized region and parallel to the longitudinal axis of the topsheet, said angle being between 10° and 40°, or between 17° to 35°, or between 20° and 35°.

The back end portion of an elasticized region (12) may also have an angle with a longitudinal line through the centre portion of the elasticized region (12) and parallel to the longitudinal axis of the topsheet, said angle being between 10° and 50°, or between 17° to 45°, or between 25° and 45°.

When both front end portions and both back end portions have an angle as above, then the elasticized regions (12a, 12b) have a so-called X-shape.

The composite sheet (10) or topsheet may have a crotch area, being the centre 30% of the topsheet, in longitudinal direction, and it may comprise one or more secondary elasticized regions in said crotch area, for example on either longitudinal side of the opening(s) (11) or part thereof, typically extending in longitudinal direction between a longitudinal side edge of the topsheet or composite sheet (10) and the elasticized region (12a, 12b) (described above) closest to said edge. Such a secondary elasticized region may have an overall curvature, curving away from the closest elasticized region, described above. Just as the elasticized region (12) described herein above, said secondary elasticized region may be patterned and it may be obtainable by the process described herein, and the description of the elastic material (15), the process of patterning and the pattern obtained with respect to the elasticized region (12) is equally applicable to the secondary elasticized area

Preferred elasticized regions (12a, 12b) of the topsheet or composite sheet (10) comprise a covering sheet, or second sheet (14), material on the side of the elastic material (15) of said region, that is not facing (and partially adhered to) the first sheet, as described above.

The longitudinal side edges of the topsheet are preferably joined or attached to the longitudinal side edges of the backsheet, by any attachment means known in the art, to form longitudinal opposing attachment areas. In one embodiment of the present invention, the topsheet and the backsheet are attached directly to one another in some locations and are indirectly joined together in other locations.

Preferably, the absorbent article of the invention is sag-tolerable, and it thereto has preferably a topsheet that is sag-tolerable as defined and described in EP1279388-A.

Any portion of the topsheet may be coated with a skin care composition or lotion or powder, known in the art. A skin care composition or lotion may be present on the elasticized regions (12a, 12b) herein, and optionally on the secondary elasticized regions. Examples of lotions include those described in U.S. 5,607,760; U.S. 5,609,587; U.S. 5,635,191; U.S. 5,643,588; WO 95/24173, provided the lotion is compatible with the elastic material (15), and does not destroy the elastic material (15) or reduce its elasticity.

Preferred absorbent articles herein comprise at least also a backsheet, an absorbent core, having a core coversheet facing the wearer in use, and preferably cuffs, such as barrier cuffs and/ or leg cuffs, which may be elasticized and comprise patterned, wrinkled elasticized regions as described herein for the composite sheet (10) herein.

The backsheet may be liquid impervious, as known in the art. In preferred embodiments, the liquid impervious backsheet comprises a thin plastic film such as a thermoplastic film having a thickness of about 0.01 mm to about 0.05 mm. Suitable backsheet materials comprise typically breathable material, which permit vapors to escape from the diaper while still preventing exudates from passing through the backsheet. Suitable backsheet films include those manufactured by Tredegar Industries Inc. of Terre Haute, IN and sold under the trade names X15306, X10962 and X10964.

The backsheet, or any portion thereof, may be elastically extendable in one or more directions. The backsheet may be attached or joined to a topsheet, the absorbent core, or any other element of the diaper by any attachment means known in the art. It may be highly preferred that the longitudinal side edges of the topsheet and backsheet are directly attached to one another, but that the longitudinal edges of the topsheet and the core are not attached to one another.

The absorbent core may comprise any absorbent material which is generally compressible, conformable, non-irritating to the wearer's skin, and capable of absorbing and retaining urine, such as comminuted wood pulp, creped cellulose wadding; melt blown polymers, including coform; chemically stiffened, modified or cross-linked cellulosic fibers; tissue, including tissue wraps and tissue laminates; absorbent foams; absorbent sponges; super absorbent polymers; absorbent gelling materials; or any other known absorbent material or combinations of materials; preferred may be absorbent cores which have an absorbent storage layer which comprises more than 80% by weight of the absorbent core content (e.g. excluding core wrap) of absorbent gelling material, and which is preferably free of airfelt.

The absorbent article may also include a sub-layer disposed between the topsheet and the absorbent core, capable of accepting, and/ or immobilizing bodily exudates, typically fecal material. Suitable materials for use as the sub-layer may include large cell open foams, macroporous compression resistant non woven highlofts, large size particulate forms of open and closed cell foams (macro and/or microporous), highloft non-wovens, polyolefin, polystyrene, polyurethane foams or particles, structures comprising a multiplicity of vertically oriented, preferably looped, strands of fibers, or preferably apertured formed films, as described above with respect to the genital coversheet. (As used herein, the term "microporous" refers to materials that are capable of transporting fluids by capillary action, but having a mean pore size of more than 50 microns. The term "macroporous" refers to materials having pores too large to effect capillary transport of fluid, generally having.pores greater than about 0.5 mm (mean) in diameter and more specifically, having pores greater than about 1.0 mm (mean) in diameter, but typically less than 10 mm or even less than 6 mm (mean).

The diapers herein may comprise a waistband, which may be an elasticized, waistband, comprising elasticized regions as described herein for the composite sheet (10) herein. The diapers herein may have a fastening system, typically joined to the waistband, as known in the art. Preferred fastening systems comprise fastening tabs and landing zones, wherein the fastening tabs are attached or joined to the back region of the diaper and the landing zones are part of the front region of the diaper.

Preferred may be that the articles of the invention (e.g. diaper) when packed in their packaging material, comprise two transverse folds, so that when unfolded for use by the user or care taker, the article (e.g. diaper) is in a U-shape and easier to apply.

### Test methods

### Handle-o-meter bending rigidity test

This method serves to determine the bending rigidity (and thereby softness) of a nonwoven layer or nonwoven sheet, as described herein, and reflects the flexibility and surface friction of the material. In this test, a nonwoven is reformed through a slot by use of a plunger, and the required force is measured. This method is based on the INDA Standard test IST 90.3-92

A sample material of the nonwoven sheet or nonwoven layer of 1 inch long and 1 inch wide (25mm x 25mm) is cut and conditioned at 65% humidity and 21°C as set out in the INDA test. The sample is free form elastic material (15) or edges attached to other materials. In one embodiment, the average fiber direction of the nonwoven web or layer in contact with the skin in use can be determined and this would be the Y direction (e.g. in use typically corresponding MD dimension of the absorbent article).

A handle-o-meter, available from Twingh- Albert Instruments Co., Philadelphia, USA, is calibrated as set out its user instructions.

The slot width is 6.35mm.

The sample is placed under the plunger and on the slot with the surface that in use contacts or faces the skin up wards facing up. A first dimension is perpendicular to the slot and this is the direction tested, for which the bending rigidity is reported herein. In one embodiment, this is the average fiber direction of the skin-facing surface, e.g. the spunbond layer. The sample is centered over the slot and the test is run and the force is measured. This value is *multiplied by 4* (e.g. normalised to a 4 inch x 4 inch sample) and reported in grams herein as the bending rigidity.

### Hydrostatic head (hydrohead)

The hydrostatic head (also referred to as hydrohead) as used herein is measured with a low surface tension liquid, i.e. a 49 mN/m liquid (solution).

This liquid is prepared as set out below.

This test is performed as set out in co-pending application WO2005/112854A, conform the Inda/ Edana test WSP 80.6 (05). However, the water pressure (from below) is increased with a rate is 60 mbar/min.

A sample of 5 cm² is taken from the composite sheet, first sheet or topsheet herein. The sample should be free from elastic material (15) or edges that are connected to other materials.

The test head used has a 2.5 cm diameter; the protective sleeve used has a 2.2 cm diameter.

The test is performed on this sample and the Hydrostatic head value is obtained, and referred to herein.

### 49 mN/m (dynes/cm) liquid preparation:

A 10 litre canister with tap is cleaned thoroughly 3 times with 2 litres polyethylene and then 3 times with 2 litres distilled/deionized water.

Then, it is filled with 10 litres distilled/deionized water and stirred with a clean stirring bar for 2 h, after which the water is released via the tap.

A 5 litre glass is cleaned 6 times with water and then 6 times with distilled/deionized water.

Then, 30.00 g of Na Cholate and 5 litres of distilled/deionized water are placed in the cleaned 5 litres glass. (NaCholate should have a TLC purity of >99%, e.g. supplied by Calbiochem, catalog # 229101). This is stirred with a clean stirring bar for about 5 min, until the Na Cholate is visibly dissolved.

The stirring bar is removed from the glass with a magnetic stick (without touching the solution) and then the Na cholate solution is poured into the 10 litres canister and more distilled/deionized water is added such that the concentration of the final solution is 3 g/l. This is further stirred with a stirring bar for 2 hours and then used.

This preparation of the solution and use thereof is at the temperature stated for the test for which it is used, or if no temperature is stated, it is kept at 20°C.

The surface tension of the solution is measured and this should be 49 mN/m (+/- 2). (The surface tension may be determined by method: ASTM D1331-56 ("Standard test method for surface and interfacial tension of solution of surface active agents") using a Kruss K12 tensiometer.)

### Strike Through value method

The low surface tension strike through value referred to herein may be obtained by the Edana method WSP70.3 (05), except that a low surface tension liquid (see below) is used and a sample of 1 inch x 1 inch (25 mm x 25 mm) may be used. The sample should be free of elastic material (15) or of edges that are connected to other materials.

The value obtained from this sample measurement is reported herein.

The low surface tension liquid is a liquid with a surface tension of 32 mN/m prepared as follows:

In a clean flask, 2.100 grams of Triton-X-100 is added to 500 ml distilled water (already in flask) and then 5000 ml distilled water is added. The solution is mixed for 30 minutes and then the surface tension is measured, which should be 32 mN/m.

(The surface tension may be determined by method: ASTM D1331-56 ("Standard test method for surface and interfacial tension of solution of surface active agents") using a Kruss K12 tensiometer.)

### Method to measure wrinkle profile/ uniformity (Primos method)

The wrinkle dimensions, e.g. height, and the wrinkle densities, and uniformity thereof, as described herein, can be measured as follows.

The composite sheet (10) with the elasticized region (12) is removed from the absorbent article such that the elongation potential, wrinkle height and wrinkle density are not changed. (If the PRIMOS equipment and method below can be used directly on the absorbent article with the composite sheet (10), then the composite sheet (10) does not need to be removed.)

It is left for 24 hours at 25°C and 50% humidity, prior to the elongation/ stretching step below, which will be performed under the same conditions.

One or more samples are marked in the partially stretched composite sheet (10), or cut there from, if necessary in order to do the PRIMOS measurement, as follows: the composite sheet (10) is gently and evenly stretched, horizontally and on a flat surface, to its fully stretched length and then released until it has 66.7% (2/3) of said fully stretched length. Then, one or more samples are marked in the composite sheet (10). The 66.7% stretched sample may be any length, but for example a sample may have a dimension of 7.5 cm in the direction of stretch (e.g. 7.5 cm of the length of the composite sheet (10) in 66.7% stretched state). A sample should have the full width of the elasticized region (12), and if possible, the full width of the composite sheet (10).

Measurement of lengths of the sample can be done with for example a micrometer screw.

The partially stretched sample, having 66.7% of its fully stretched length (for example a sample of 7.5cm) is then examined by use of PRIMOS equipment and its data acquisition software, following the manufacture's instructions manual, using a 13x18mm lens.

The PRIMOS equipment and software will measure all peak heights, widths etc and the herein described values can be calculated there from. The height is the distance between the highest and lowest point of a peak. It should be noted that "shoulder peaks" are not regarded peaks of the wrinkles herein, as is a known approach in the art. Namely, if two adjacent peaks, A and B, are joined by a valley and either a) the distance from the highest point of peak A to the lowest point of the valley is less than 30% of the height of peak A *or b)* the distance from the highest point of peak B to the lowest point of the valley is less than 30% of the height of peak B, then said peak B is considered a shoulder peak and not an individual peak, and thus peak A and B are considered a single peak A (e.g. with a single width, single height etc.). Thus, if either a) or b) above applies, A and B are taken as one single peak.

### Residual strain

The residual strain of an absorbent article or of a composite sheet (10), obtainable by the process herein can be calculated as follows.

The elastic material (15) is conditioned as above. The contracted, relaxed length of the elastic material (15) or elasticized region (12) as used in the process to form the composite sheet (10) herein is determined. This is l₀.

Then, the length of the elastic material (15) or elasticized region (12) in the contracted absorbent article or composite sheet (10), conditioned as set out in the method above and on a flat surface, is measured. This is lₓ.

Then, the residual strain Sᵣ = [(lₓ - l₀)/ lₓ] can be calculated.

For example, if 10 cm elastic material (15) is attached to a first sheet (13) over a length of 40 cm, and the resulting composite sheet (10) has a contracted length of 15 cm, then the residual strain is (15-10)/ 15 = 33.3%.

In a finished article, the residual strain can be calculated if the elastic material (15) can be removed from the article and then, the contracted length thereof can be calculated as above.

### (This of course after having calculated lₓ)

### Peel force method

This method serves to determine the strength of the bond in the composite sheet (10) of the elastic material (15) and the first sheet (13 and second sheet (14); the herein reported peel force is the force required to undo the bond of (delaminate) the elastic and the sheets.

The measurement may be done with for example a Zwick 2.5 KN tensile tester with a load cell of 50N. The test path is 100 mm. The speed is set to be 100 mm/ min. The clamps are for example 25mm x 40 mm. the target gage length can suitably be set, e.g. 25 mm. (N.B.: In general, he load cell should be chosen in a way that the expected measurement values are in the calibrated range of the capacity of the load cell (e.g. 0.2-100% of capacity, which is for a 50 N load cell from 0.1 N - 50 N)).

A sample is cut from the composite sheet (10) such that the whole width of the elasticized region (12) (and of the elastic material (15)) is comprised in the sample and such that at one longitudinal side (in direction of stretch) an area is present that is formed by the first and second sheet material, but not by the elastic material.

A JDC precision Sample cutter by Thwings-Albert Instrument Company , USA, may be used

A suitable sample may be 1 inch (25.4) long (in direction of stretch, Y-direction) and having the width of the elastic material (15) plus along one longitudinal side of the sample some of the neighboring first sheet (13) and second sheet that is not part of the elasticized region (12), e.g. attached to said elastic material (15), e.g. 40 mm.

The sample is conditions for 16 hours at 50% relative humidity and 20°C.

Then the area formed by the first sheet and second sheet is carefully peeled open up to the elastic material; the peeled open area of the first sheet and second sheet are attached between the clamps of the test equipment, so that there is no slag in the sample. The test can then be run and the force to peel the elastic material and the first sheet and/ or second sheet is recorded and reported as peel force value used herein. If the composite sheet allows more samples to be taken, then this test can be repeated for more samples, and the average peel force can be obtained, and reported herein as peel force values.

## Claims

1. A process for making a composite sheet (10) useful for an absorbent article, said composite sheet (10) comprising a wrinkled, patterned elasticized region (12a or 12b) that comprises a patterned first sheet (13) and an elastic material (15), said composite sheet (10) being obtainable by:
a. obtaining a first sheet (13) with a longitudinal direction Y and a transverse direction X;
b. obtaining an elastic material (15) that is at least partially stretched;
c. positioning said at least partially stretched elastic material (15) adjacent said first sheet (13) to obtain a combined material and simultaneously submitting the combined material, or part thereof, to a patterning, pressure-applying step to obtain a patterned combined material, comprising a patterned first sheet (13) comprising troughs (16);
d) simultaneously to step c) attach the thus formed troughs (16), or part thereof, of the patterned first sheet (13) to said elastic material (15), to thus obtain a stretched composite sheet (10) comprising a patterned elasticized region;
e) relaxing the composite sheet (10) of step d) to obtain a composite sheet (10) comprising a wrinkled, patterned elasticized region (12), comprising wrinkles with peaks (21) and valleys (22),
said composite sheet (10) comprising one or more openings to receive fecal material and comprising said step c whereby said first sheet (13) and elastic material (15) are positioned adjacent one another prior to or simultaneously with said patterning, pressurizing step.

2. A process as in claim 1, whereby in step c) a first surface of said first sheet (13) or part thereof, that is not to face or not facing the elastic material (15), is pressurized, and optionally contacted, by a first surface (31) of a first tool (30), having a multitude of raised portions (32), whereby said raised portions form said troughs (16) in said first sheet (13) or part thereof; and whereby a second surface of said first sheet, facing the elastic material (15) is pressurized, and optionally indirectly contacted, by a second, non-mating surface (35) of a second tool (34).

3. A process as in claim 2, whereby said first tool (30) has a transverse axis (33) and whereby said raised portions (32) are in the form of: a) a multitude of ridges, each ridge being positioned under angle of between 0[deg.] to 45[deg.] with a line parallel to the transverse axis (33), and/ or b) a multitude of teeth and/ or studs, positioned along the X and Y direction of said tool's surface, in first rows positioned under angle of between 0[deg.] to 45[deg.] with a line parallel to the transverse axis (33) and second rows positioned perpendicular to said first rows.

4. A process as in claim 2, whereby the distance between said raised portions (32) and the second tool's surface (35) is 0.01 mm to 0.25 mm.

5. A process as in claim 2, whereby said raised portions include a first flat surface (in x-y plane) that contacts the sheet material first, said first surface having a Y-dimension (substantially perpendicular to the X-direction of the tool.) of from 0.05 mm to 0.5 mm, or preferably from 0.1 mm or 0.2 mm to 0.4 mm, and whereby when the raised portions are studs or teeth, having an X-dimension of from 0.05 mm to 2.0 mm, or preferably from 0.1 mm or 0.2 mm to 0.4 mm.

6. An absorbent article comprising a composite sheet (10) obtained by the process of claim 1, whereby the composite sheet (10) comprises one or more openings (11) to receive fecal material; a wrinkled, patterned elasticized region; a longitudinal direction Y and a transverse direction X; and a first sheet (13) and an elastic material (15), said elastic material (15) extending in longitudinal direction and providing elasticity in said longitudinal direction, whereby said first sheet, or part thereof, comprises in longitudinal direction, and optionally in transverse direction, a pattern of a multitude of troughs (16) and a multitude of crests (17), said troughs (16) of said first sheet (13) having a higher density than the crests (17) of said first sheet; and whereby said elastic material (15) is attached to said troughs (16) of said first sheet.

7. An absorbent article as in claim 6, whereby said composite sheet (10) is a composite barrier sheet, having a hydrostatic head of at least 15 mbar and/ or said first sheet (13) is a barrier sheet having a hydrostatic head of at least 15 mbar, as measured using Inda/Edana test WSP 80.6 (05) with a water pressure from below increased to a rate of 60 mbar/min as further detailed in the description herein.

8. An absorbent article as in claim 6, whereby said first sheet (13) is a nonwoven sheet or nonwoven layer, having at least two spunbond webs and at least one meltblown web, said sheet or layer having a basis weight of at least 17 g/m², and said sheet or layer comprising at least 3 g/m² meltblown fibers.

9. An absorbent article as in claim 6, whereby said elasticized region (12) comprises adhesive filaments, having an average diameter of less than 200 microns, adhering the elastic material (15) and said troughs (16) of the first sheet.

## Patentansprüche

1. Verfahren zum Herstellen einer Verbundstofflage (10), die für einen Absorptionsartikel geeignet ist, wobei die Verbundstofflage (10) einen faltigen, gemusterten elastifizierten Bereich (12a oder 12b) umfasst, der eine gemusterte erste Lage (13) und ein elastisches Material (15) umfasst, wobei die Verbundstofflage (10) hergestellt wird durch:
a. Herstellen einer ersten Lage (13) mit einer Längsrichtung Y und einer Querrichtung X;
b. Herstellen eines elastischen Materials (15), das mindestens teilweise gedehnt ist;
c. Positionieren von mindestens dem teilweise gedehnten elastischen Material (15) angrenzend an die erste Lage (13), um ein kombiniertes Material zu erhalten, und gleichzeitiges Anwenden eines Muster prägenden, Druck anwendenden Schritts auf das kombinierte Material oder einen Teil davon, um ein gemustertes kombiniertes Material zu erhalten, das eine gemusterte erste Lage (13) umfasst, die Mulden (16) umfasst;
d) gleichzeitig mit Schritt c) Befestigen der so gebildeten Mulden (16), oder eines Teils davon, der gemusterten ersten Lage (13) an dem elastischen Material (15), um so eine gedehnte Verbundstofflage (10) zu erhalten, die einen gemusterten elastifizierten Bereich umfasst;
e) Entspannen der Verbundstofflage (10) von Schritt d), um eine Verbundstofflage (10) zu erhalten, die einen faltigen, gemusterten elastifizierten Bereich (12) umfasst, der Falten mit Erhebungen (21) und Vertiefungen (22) umfasst,
wobei die Verbundstofflage (10) eine oder mehrere Öffnungen umfasst, um Fäkalmaterial aufzunehmen, und umfassend den Schritt c, wobei die erste Lage (13) und das elastische Material (15) vor oder gleichzeitig mit dem Muster prägenden, Druck anwendenden Schritt aneinander angrenzend angeordnet werden.

2. Verfahren nach Anspruch 1, wobei in Schritt c) eine erste Oberfläche der ersten Lage (13) oder ein Teil davon, die bzw. der nicht zu dem elastischen Material (15) weisen soll oder weist, von einer ersten Oberfläche (31) eines ersten Werkzeugs (30), das eine Vielzahl erhöhter Abschnitte (32) aufweist, wobei die erhöhten Abschnitte die Mulden (16) in der ersten Lage (13) oder einem Teil davon bilden, druckbeaufschlagt und wahlweise berührt wird; und wobei eine zweite Oberfläche der ersten Lage, die zu dem elastischen Material (15) weist, von einer zweiten Oberfläche (35) eines zweiten Werkzeugs (34), die keine Passoberfläche ist, druckbeaufschlagt und wahlweise indirekt berührt wird.

3. Verfahren nach Anspruch 2, wobei das erste Werkzeug (30) eine Querachse (33) aufweist und wobei die erhöhten Abschnitte (32) folgende Form aufweisen: a) eine Vielzahl von Erhebungen, wobei jede Erhebung unter einem Winkel zwischen 0° bis 45° mit einer Linie parallel zur Querachse (33) angeordnet ist, und/oder b) eine Vielzahl von Zähnen und/oder Zapfen, die entlang der X- und Y-Richtung der Werkzeugoberfläche, in ersten Reihen, die unter einem Winkel zwischen 0° bis 45° mit einer Linie parallel zur Querachse (33) angeordnet sind, und in zweiten Reihen, die senkrecht zu den ersten Reihen angeordnet sind, positioniert sind.

4. Verfahren nach Anspruch 2, wobei der Abstand zwischen den erhöhten Abschnitten (32) und der zweiten Werkzeugoberfläche (35) 0,01 mm bis 0,25 mm beträgt.

5. Verfahren nach Anspruch 2, wobei die erhöhten Abschnitte eine erste flache Oberfläche (in X-Y-Ebene) aufweist, die das Lagenmaterial zuerst berührt, wobei die erste Oberfläche eine Y-Abmessung (im Wesentlichen senkrecht zur X-Richtung des Werkzeugs) von 0,05 mm bis 0,5 mm oder vorzugsweise von 0,1 mm oder 0,2 mm bis 0,4 mm aufweist und wobei, wenn die erhöhten Abschnitte Zapfen oder Zähne sind, eine X-Abmessung von 0,05 mm bis 2,0 mm oder vorzugsweise von 0,1 mm oder 0,2 mm bis 0,4 mm beträgt.

6. Absorptionsartikel, umfassend eine Verbundstofflage (10), die gemäß dem Verfahren nach Anspruch 1 hergestellt wird, wobei die Verbundstofflage (10) eine oder mehrere Öffnungen (11) zum Aufnehmen von Fäkalmaterial umfasst; einen faltigen, gemusterten elastifizierten Bereich; eine Längsrichtung Y und eine Querrichtung X; und eine erste Lage (13) und ein elastisches Material (15), wobei das elastische Material (15) in Längsrichtung verläuft und Elastizität in der Längsrichtung verleiht, wobei die erste Lage, oder ein Teil davon, in Längsrichtung und wahlweise in Querrichtung ein Muster einer Vielzahl von Mulden (16) und einer Vielzahl von Erhebungen (17) umfasst, wobei die Mulden (16) der ersten Lage (13) eine höhere Dichte aufweisen als die Erhebungen (17) der ersten Lage; und wobei das elastische Material (15) an den Mulden (16) der ersten Lage angebracht ist.

7. Absorptionsartikel nach Anspruch 6, wobei die Verbundstofflage (10) eine Verbundstoff-Isolierschicht mit einer Wassersäule von mindestens 1,5 kPa (15 mbar) ist und/oder die erste Lage (13) eine Isolierschicht mit einer Wassersäule von mindestens 1,5 kPa (15 mbar) ist, wie mit dem Inda-/Edana-Test WSP 80.6 (05) mit einem Wasserdruck gemessen, der von unten um eine Rate von 6 kPa/min (60 mbar/min) erhöht wird, wie in der Beschreibung hierin ausführlich beschrieben ist.

8. Absorptionsartikel nach Anspruch 6, wobei die erste Lage (13) eine Vlieslage oder Vliesschicht ist, die mindestens zwei Spinnvliesbahnen und mindestens eine schmelzgeblasene Bahn aufweist, wobei die Lage oder Schicht ein Flächengewicht von mindestens 17 g/ m² aufweist und die Lage oder Schicht mindestens 3 g/m² schmelzgeblasene Fasern aufweist.

9. Absorptionsartikel nach Anspruch 6, wobei der elastifizierte Bereich (12) Klebstofffilamente mit einem durchschnittlichen Durchmesser von weniger als 200 Mikrometern umfasst, die das elastische Material (15) und die Mulden (16) der ersten Lage kleben.

## Revendications

1. Procédé de fabrication d'une feuille composite (10) utile pour un article absorbant, ladite feuille composite (10) comprenant une région élastifiée froissée à motifs (12a ou 12b) qui comprend une première feuille à motifs (13) et un matériau élastique (15), ladite feuille composite (10) étant obtenue en :
a. obtenant une première feuille (13) avec une direction longitudinale Y et une direction transversale X ;
b. obtenant un matériau élastique (15) qui est au moins partiellement étiré ;
c. positionnant ledit au moins un matériau élastique partiellement étiré (15) adjacent à ladite première feuille (13) pour obtenir un matériau combiné et en soumettant simultanément le matériau combiné, ou une partie de celui-ci, à une étape de formation de motif par application de pression pour obtenir un matériau combiné à motifs, comprenant une première feuille à motifs (13) comprenant des creux (16) ;
d) fixant simultanément à l'étape c) les creux ainsi formés (16), ou une partie de ceux-ci, de la première feuille à motifs (13) audit matériau élastique (15), pour obtenir ainsi une feuille composite étirée (10) comprenant une région élastifiée à motifs ;
e) relâchant la feuille composite (10) de l'étape d) pour obtenir une feuille composite (10) comprenant une région élastifiée froissée à motifs (12), comprenant des plis avec des pics (21) et des vallées (22),
ladite feuille composite (10) comprenant une ou plusieurs ouvertures pour recevoir des matières fécales et comprenant ladite étape c selon laquelle ladite première feuille (13) et le matériau élastique (15) sont positionnés l'un à côté de l'autre avant ou en même temps que ladite étape de formation de motif sous pression.

2. Procédé selon la revendication 1, selon lequel dans l'étape c) une première surface de ladite première feuille (13) ou une partie de celle-ci, qui n'est pas en face ou ne fait pas face au matériau élastique (15), est mise sous pression, et facultativement mise en contact, par une première surface (31) d'un premier outil (30), ayant une multitude de parties relevées (32), selon quoi lesdites parties relevées forment lesdits creux (16) dans ladite première feuille (13) ou une partie de celle-ci ; et selon quoi une deuxième surface de ladite première feuille, faisant face au matériau élastique (15) est mise sous pression, et facultativement mise indirectement en contact, par une deuxième surface sans appariement (35) d'un deuxième outil (34).

3. Procédé selon la revendication 2, selon lequel ledit premier outil (30) a un axe transversal (33) et selon lequel lesdites parties relevées (32) sont sous la forme de :
a) une multitude de crêtes, chaque crête étant positionnée sous un angle compris entre 0° et 45° avec une ligne parallèle à l'axe transversal (33), et/ou b) une multitude de dents et/ou plots, positionnés le long des directions X et Y de la surface dudit outil, dans des premières rangées positionnées sous un angle compris entre 0° et 45° avec une ligne parallèle à l'axe transversal (33) et des deuxièmes rangées positionnées perpendiculaires auxdites premières rangées.

4. Procédé selon la revendication 2, selon lequel la distance entre lesdites parties relevées (32) et la surface du deuxième outil (35) va de 0,01 mm à 0,25 mm.

5. Procédé selon la revendication 2, selon lequel lesdites parties relevées incluent une première surface plate (dans le plan x-y) qui vient d'abord en contact avec le matériau de feuille, ladite première surface ayant une dimension Y (essentiellement perpendiculaire à la direction X de l'outil) allant de 0,05 mm à 0,5 mm, ou de préférence de 0,1 mm ou 0,2 mm à 0,4 mm, et selon lequel, lorsque les parties relevées sont des plots ou des dents, ayant une dimension X allant de 0,05 mm à 2,0 mm, ou de préférence de 0,1 mm ou 0,2 mm à 0,4 mm.

6. Article absorbant comprenant une feuille composite (10) obtenue par le procédé selon la revendication 1, selon lequel la feuille composite (10) comprend une ou plusieurs ouvertures (11) pour recevoir les matières fécales ; une région élastifiée froissée à motifs ; une direction longitudinale Y et une direction transversale X ; et une première feuille (13) et un matériau élastique (15), ledit matériau élastique (15) s'étendant dans la direction longitudinale et fournissant une élasticité dans ladite direction longitudinale, selon quoi ladite première feuille, ou une partie de celle-ci, comprend dans la direction longitudinale, et facultativement dans la direction transversale, un motif d'une multitude de creux (16) et une multitude de crêtes (17), lesdits creux (16) de ladite première feuille (13) ayant une densité plus élevée que les crêtes (17) de ladite première feuille ; et selon quoi ledit matériau élastique (15) est fixé auxdits creux (16) de ladite première feuille.

7. Article absorbant selon la revendication 6, selon lequel ladite feuille composite (10) est une feuille formant barrière composite, ayant une charge hydrostatique d'au moins 1,5 kPa (15 mbar) et/ou ladite première feuille (13) est une feuille formant barrière ayant une charge hydrostatique d'au moins 1,5 kPa (15 mbar), telle que mesurée en utilisant le test Inda/Edana WSP 80.6 (05) avec une pression d'eau par le bas augmentée à un taux de 6 kPa/min (60 mbar/min) comme détaillé davantage dans la présente description.

8. Article absorbant selon la revendication 6, selon lequel ladite première feuille (13) est une feuille non tissée ou une couche non tissée, ayant au moins deux nappes filées-liées et au moins une nappe soufflée en fusion, ladite feuille ou couche ayant une masse surfacique d'au moins 17 g/m², et ladite feuille ou couche comprenant au moins 3 g/m² de fibres soufflées en fusion.

9. Article absorbant selon la revendication 6, selon lequel ladite région élastifiée (12) comprend des filaments adhésifs; ayant un diamètre moyen inférieur à 200 microns, faisant adhérer le matériau élastique (15) et lesdits creux (16) de la première feuille.
